# EUROPÄISCHE PATENTSCHRIFT

(11) **EP 2 513 177 B1**
(45) Veröffentlichungstag und Bekanntmachung des Hinweises auf die Patenterteilung: **10.12.2014**
(21) Anmeldenummer: 10790432.8
(22) Anmeldetag: 10.12.2010
(51) Int. Cl.: C08G 18/08, C08G 18/12, C08G 18/44, C09D 175/04, C09D 175/06, A61L 29/08, A61L 31/10

(54) **POLYURETHANHARNSTOFF FÜR STENTBESCHICHTUNGEN**
POLYURETHANE UREA FOR STENT COATINGS
URÉE DE POLYURÉTHANE POUR REVÊTEMENTS DE STENTS

(30) Priorität: 16.12.2009 EP 09015532
(43) Veröffentlichungstag der Anmeldung: 24.10.2012
(73) Patentinhaber: Bayer Intellectual Property GmbH, 40789 Monheim am Rhein (DE)
(72) Erfinder: KÖCHER, Jürgen, 40764 Langenfeld (DE); WAMPRECHT, Christian, 41472 Neuss (DE); ROHM, Henning, 53225 Bonn (DE); SCHMITZ, Klaus-Peter, 18119 Rostock-Warnemünde (DE); STERNBERG, Katrin, 18055 Rostock (DE)
(86) Internationale Anmeldenummer: PCT/EP2010/069393
(87) Internationale Veröffentlichungsnummer: WO 2011/082946

(56) Entgegenhaltungen:
- WO-A1-2009/012391

## Beschreibung

Die Erfindung betrifft einen Polyurethanharnstoff, der insbesondere zur Herstellung von Stentbeschichtungen eingesetzt werden kann. Ein weiterer Gegenstand der Erfindung ist ein Substrat mit einer Grundbeschichtung aus einem erfindungsgemäßen Polyurethanharnstoff. Ebenfalls Gegenstand der Erfindung ist ein Schichtaufbau umfassend wenigstens eine wirkstoffhaltige Schicht aus einem erfindungsgemäßen Polyurethanharnstoff und wenigstens eine wirkstofffreie Schicht aus einem erfindungsgemäßen Polyurethanharnstoff. Schließlich ist auch ein Verfahren zum Beschichten eines Substrats, bei dem auf das Substrat wenigstens eine Schicht aus einem erfindungsgemäßen Polyurethanharnstoff aufgebracht wird, Gegenstand der Erfindung.

Im Stand der Technik sind Polymer-basierte Beschichtungen für implantierbare Artikel wie Stents bekannt. Diese enthalten häufig Wirkstoffe wie Paclitaxel oder Sirolimus, wobei die Beschichtungen dafür ausgelegt sind, diese Wirkstoffe über einen längeren Zeitraum freizusetzen, wenn der Stent in einem Körper implantiert ist. Durch die verzögerte Wirkstoffabgabe soll insbesondere die Gefahr der Restenose des behandelten Gefäßes verringert werden.

Ein derartiger beschichteter Stent ist beispielsweise in der DE 10 2005 010 998 A1 beschrieben. Hier wird eine wirkstoffhaltige Beschichtung aus einem Polyurethanharnstoff vorgeschlagen. Es hat sich allerdings gezeigt, dass die Abgabe des Wirkstoffs aus der Polyurethanhamstoff-Beschichtung zu schnell erfolgt. So wird zu Beginn der Freisetzung (unmittelbar nach Implantation) eine zu große Menge Wirkstoff pro Zeit abgegeben wohingegen am Ende der Gesamtfreisetzungsdauer zu geringe Konzentrationen des Wirkstoffs freigesetzt werden. Darüber hinaus ist die Gesamtabgabedauer an Wirkstoff zu kurz.

In der WO 2009/115264 A1 ist ebenfalls ein wirkstoffhaltiger Polyurethanharnstoff offenbart, der zur Herstellung von Beschichtungen auf Stents verwendet werden kann. Diese Polyurethanstoff-Beschichtungen zeichnen sich durch eine gute Biokompatibilitäl aus. Jedoch zeigen auch Stents, die mit dieser Beschichtung versehen sind, grundsätzlich die bereits für die DE 10 2005 010 998 A1 beschriebene Freisetzungskinetik, d.h. es wird insbesondere zu Beginn der Freisetzung eine zu große Menge an Wirkstoff aus der Beschichtung freigegeben.

Wirkstoffhaltige Polyurethanharnstoff-Beschichtungen für Stents sind auch aus den beiden noch nicht veröffentlichten PCT-Anmeldungen mit den Anmeldenummern PCT/EP2009/006101 und PCT/EP2009/006102 bekannt. Die hier beschriebenen Polyurethanharnstoffe sind jeweils mit einer Copolymereinheit aus Polyethylenoxid und Polypropylenoxid terminiert.

Die im Stand der Technik bekannten Polymer-basierten wirkstoffhaltigen Stentbeschichtungen setzen den enthaltenen Wirkstoff zu schnell und in zu hoher anfänglicher Konzentration frei. Dies hat vor allem zur Folge, dass der Wirkstoff nicht über die idealerweise anzustrebende Abgabedauer von 4 bis 12 Wochen in der nötigen Konzentration zur Verfügung steht.

Aufgabe der Erfindung war es daher, einen Polyurethanharnstoff bereit zu stellen, der insbesondere zur Herstellung von wirkstoffhaltigen Beschichtungen für Stents geeignet ist, die den Wirkstoff nach Implantation mit einer gleichmäßigen Abgabegeschwindigkeit über einen Zeitraum von 4 bis 12 Wochen freisetzen.

Diese Aufgabe ist durch einen Polyurethanharnstoff gelöst, der Struktureinheiten der Formel (I) 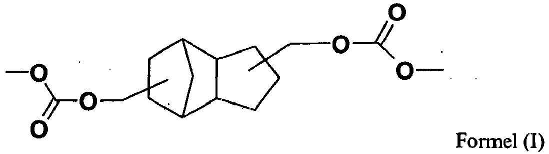 aufweist und nicht mit wenigstens einer Copolymereinheit aus Polyethylenoxid und Polypropylenoxid tenniniert ist.

Polyurethanharnstoffe im Sinne der vorliegenden Erfindung sind polymere Verbindungen, die
(a) mindestens zwei Urethangruppen enthaltende Wiederholungseinheiten der folgenden allgemeinen Struktur 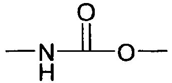 und
(b) mindestens eine Harnstoffgruppen enthaltende Wiederholungseinheit 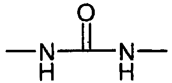 aufweisen.

Das zahlenmittlere Molekulargewicht der Polyurethanharnstoffe beträgt vorzugsweise 1000 bis 200000 g/mol, besonders bevorzugt von 3000 bis 100000 g/mol. Dabei wird das zahlenmittlere Molekulargewicht gegen Polystyrol als Standard in Dimethylacetamid bei 30 °C gemessen.

Die erfindungsgemäßen Polyurethanharnstoffe können durch Umsetzung von Komponenten hergestellt werden, die mindestens eine Polycarbonatpolyolkomponente a), mindestens eine Polyisocyanatkomponente b), mindestens eine Diamin- und/oder Aminoalkoholkomponente c) und gegebenenfalls eine weitere Polyolkomponente d) umfassen.

Gemäß einer bevorzugten Ausführungsform der Erfindung ist vorgesehen, dass der Polyurethanharnstoff auf einer Polycarbonatpolyolkomponente basiert, die bevorzugt eine mittlere Hydroxylfunktionalität von 1,7 bis 2,3 aufweist.

Die Polyurethanharnstoffe sind bevorzugt im Wesentlichen lineare Moleküle, können jedoch auch verzweigt sein, was jedoch weniger bevorzugt ist. Unter im Wesentlichen linearen Molekülen versteht man im Rahmen der vorliegenden Erfindung leicht anvernetzte Systeme, wobei die zugrundeliegende Polycarbonalpolyolkomponente a) eine mittlere Hydroxylfunktionalität von bevorzugt 1,7 bis 2,3, besonders bevorzugt 1,8 bis 2,2, ganz besonders bevorzugt 1,9 bis 2,1, aufweisen kann.

Die Polycarbonatpolyolkomponente a) kann Polycarbonatpolyole a1) umfassen, die durch Umsetzung von Kohlensäurederivaten mit difunktionellen Alkoholen der Formel (II) 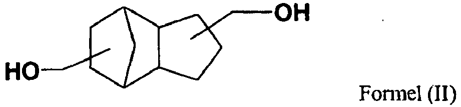 erhältlich sind.

Zur Herstellung des Polycarbonatpolyols a1) kann in einem Druckreaktor bei erhöhter Temperatur TCD Alkohol DM [3(4),8(9)-Bis(hydroxymethyl)-tricyclo(5.2.1.0/2.6)decan/ Tricyclodecandimethanol] mit Diphenylcarbonat, Dimethylcarbonat oder Phosgen umgesetzt werden. Bevorzugt ist die Umsetzung mit Dimethylcarbonat. Im Falle der Verwendung von Dimethylcarbonat wird das Spaltprodukt Methanol im Gemisch mit überzähligem Dimethylcarbonat per Destillation entfernt.

Die Polycarbonatpolyole a1) auf Basis von Diolen der Formel (II) haben vorzugsweise durch die OH-Zahl bestimmte Molekulargewichte von 200 bis 10000 g/mol, besonders bevorzugt von 300 bis 8000 g/mol und ganz besonders bevorzugt von 400 bis 6000 g/mol.

Zusätzlich kann die Polycarbonatpolyolkomponente a) neben den Polycarbonatpolyolen a1) weitere Polycarbonatpolyole a2) umfassen.

Bei den Polycarbonatpolyolen a2) kann es sich bevorzugt um Verbindungen mit einer mittleren Hydroxylfunktionalität von 1,7 bis 2,3 und einem durch die OH-Zahl bestimmtem Molekulargewicht von 400 bis 6000 g/mol auf Basis von Hexandiol-1,6, Butandiol-1,4 oder deren Mischungen handln.

Ferner weisen die Polycarbonatpolyole a2) vorzugsweise durch die OH-Zahl bestimmte Molekulargewichte von 400 bis 6000 g/mol, besonders bevorzugt von 500 bis 5000 g/mol, ganz besonders bevorzugt von 600 bis 3000 g/mol auf. Sie sind beispielsweise durch Reaktion von Kohlensäurederivaten, wie Diphenylcarbonat, Dimethylcarbonat oder Phosgen, mit Polyolen, vorzugsweise Diolen, erhältlich. Als Diole kommen dabei beispielsweise Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentan-1,3-diol, Di-, Tri- oder Tetraethylenglykol, Dipropylenglykol, Polypropylenglykole, Dibutylenglykol, Polybutylenglykole, Bisphenol A, Tetrabrombisphenol A aber auch Lacton-modifizierte Diole in Frage.

Die Polycarbonatpolyole a2) enthalten vorzugsweise 40 bis 100 Gew.-% Hexandiol, bevorzugt 1,6-Hexandiol und/oder Hexandiol-Derivate. Sie enthalten vorzugsweise solche Derivate, die neben endständigen OH-Gruppen Ether- oder Estergruppen aufweisen. Dies sind z.B. Produkte, die durch Umsetzung von 1 Mol Hexandiol mit mindestens 1 Mol, bevorzugt 1 bis 2 Mol Caprolacton oder durch Veretherung von Hexandiol mit sich selbst zum Di- oder Trihexylenglykol erhalten werden können. Auch Polyether-Polycarbonatdiole können eingesetzt werden. Die Hydroxylpolycarbonate können insbesondere im wesentlichen linear sein. Sie können jedoch gegebenenfalls durch den Einbau polyfunktioneller Komponenten, insbesondere niedermolekularer Polyole, leicht verzweigt werden. Hierzu eignen sich beispielsweise Glycerin, Hexantriol-1,2,6, Butantriol-1,2,4, Trimethylolpropan, Pentaerythrit, Chinit, Mannit, Sorbit, Methylglykosid oder 1,3,4,6-Dianhydrohexite. Bevorzugt sind solche Polycarbonatpolyole a2) auf Basis von Hexandiol-1,6, sowie modifizierend wirkenden Co-Diolen wie z. B. Butandiol-1,4 oder auch von ε-Caprolacton. Weitere bevorzugte Polycarbonatpolyole a2) sind solche auf Basis von Mischungen aus Hexandiol-1,6 und Butandiol-1,4.

In einer bevorzugten Ausführungsform wird als Polycarbonatpolyolkomponente a) eine Mischung aus den Polycarbonatpolyolen a1) und solchen Polycarbonatpolyolen a2) auf Basis von Hexandiol-1,6, Butandiol-1,4 oder deren Mischungen eingesetzt.

Im Fall von Mischungen der Polycarbonatpolyole a1) und a2) beträgt der Anteil von a1) an der Mischung bevorzugt mindestens 5 mol%, besonders bevorzugt mindestens 10 mol%, bezogen auf die gesamte Molmenge an Polycarbonatpolyol.

Die Polyurethanharnstoffe können ferner Einheiten aufweisen, die auf mindestens ein Polyisocyanat als Aufbaukomponente b) zurückgehen.

Als Polyisocyanate b) können alle dem Fachmann bekannten aromatischen, araliphatischen, aliphatischen und cycloaliphatischen Isocyanate einer mittleren NCO-Funktionalität ≥ 1, bevorzugt ≥ 2 einzeln oder in beliebigen Mischungen untereinander eingesetzt werden, wobei es unerheblich ist, ob diese nach Phosgen- oder Phosgen-freien Verfahren hergestellt wurden. Diese können auch Iminooxadiazindion-, Isocyanurat-, Uretdion-, Urethan-, Allophanat-, Biuret-, Harnstoff-, Oxadiazintrion, Oxazolidinon-, Acylharnstoff- und/oder Carbodiimid-Strukturen aufweisen. Die Polyisocyanate können einzeln oder in beliebigen Mischungen untereinander eingesetzt werden.

Bevorzugt werden Isocyanate aus der Reihe der aliphatischen oder cycloaliphatischen Vertreter eingesetzt, wobei diese ein Kohlenstoffgrundgerüst (ohne die enthaltenen NCO-Gruppen) von 3 bis 30, bevorzugt 4 bis 20 Kohlenstoffatomen aufweisen.

Besonders bevorzugte Verbindungen der Komponente b) entsprechen der vorstehend genannten Art mit aliphatisch und/oder cycloaliphatisch gebundene NCO-Gruppen wie beispielsweise Bis-(isocyanatoalkyl)ether, Bis- und Tris-(isocyanatoalkyl)benzole, -toluole, sowie -xylole, Propandiisocyanate, Butandiisocyanate, Pentandiisocyanate, Hexandiisocyanate (z.B. Hexamethylendiisocyanat, HDI), Heptandiisocyanate, Octandiisocyanate, Nonandiisocyanate (z.B. Trimethyl-HDI (TMDI) in der Regel als Gemisch der 2,4,4- und 2,2,4-Isomeren), Nonantriisocyanate (z.B. 4-Isocyanatomethyl-1,8-octandiisocyanat), Dekandiisocyanate, Dekantriisocyanate, Undekandiisocyanate, Undekantriisocyanate, Dodecandiisocyanate, Dodecantriisocyanate, 1,3- sowie 1,4-Bis-(isocyanatomethyl)cyclohexane (H₆XDI), 3-Isocyanatomethyl-3,5,5-trimethylcyclohexylisocyanat (Isophorondiisocyanat, IPDI), Bis-(4-isocyanatocyclohexyl)methan (H₁₂MDI) oder Bis-(isocyanatomethyl)norbornan (NBDI).

Ganz besonders bevorzugte Verbindungen der Komponente b) sind Hexamethylendiisocyanat (HDI), Trimethyl-HDI (TMDI), 2-Methylpentan-1,5-diisocyanat (MPDI), Isophorondiisocyanat (IPDI), 1,3- sowie 1,4-Bis(isocyanatomethyl)cyclohexan (H₆XDI), Bis(isocyanatomethyl)-norbornan (NBDI), 3(4)-Isocyanatomethyl-1-methyl-cyclohexylisocyanat (IMCI) und/oder 4,4'-Bis(isocyanatocyclohexyl)methan (H₁₂MDI) oder Gemische dieser Isocyanate. Weitere Beispiele sind Derivate aus den vorstehenden Diisocyanaten mit Uretdion-, Isocyanurat-, Urethan-, Allophanat-, Biuret-, Iminooxadiazindion- und/oder Oxadiazintrionstruktur mit mehr als zwei NCO-Gruppen.

Die Menge an Polyisocyanaten b) bei der Herstellung der Polyurethanharnstoffe beträgt vorzugsweise 1.0 bis 3.5 mol, besonders bevorzugt 1,0 bis 3,3 mol und ganz besonders bevorzugt 1,0 bis 3,0 mol. jeweils bezogen auf die Menge der Verbindungen der Polycarbonatpolyolkomponente a).

Die Polyurethanharnstoffe können Einheiten aufweisen, die auf mindestens ein Diamin oder ein Aminoalkohol als Aufbaukomponente zurückgehen und als Kettenverlängerer c) dienen.

Solche Kettenverlängerer c) sind beispielsweise Di- oder Polyamine sowie Hydrazide, z.B. Hydrazin, Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 1,3- und 1,4-Xylylendiamin, α,α,α',α'-Tetramethyl-1,3- und -1,4-xylylendiamin und 4,4'-Diaminodicyclohexylmethan, Dimethylethylendiamin, Adipinsäuredihydrazid, 1,4-Bis(aminomethyl)cyclohexan, 4,4'-Diamino-3,3'-dimethyldicyclohexyl-methan und andere (C₁-C₄)-Di- und Tetraalkyldicyclo-hexylmethane, z. B. 4,4'-Diamino-3,5-diethyl-3',5'-diisopropyldicyclohexylmethan.

Als Diamine oder Aminoalkohole kommen im Allgemeinen niedermolekulare Diamine oder Aminoalkohole in Betracht, die aktiven Wasserstoff mit gegenüber NCO-Gruppen unterschiedlicher Reaktivität enthalten, wie Verbindungen, die neben einer primären Aminogruppe auch sekundäre Aminogruppen oder neben einer Aminogruppe (primär oder sekundär) auch OH-Gruppen aufweisen. Beispiele hierfür sind primäre und sekundäre Amine, wie 3-Amino-1-Methylaminopropan, 3-Amino-1-Ethylaminopropan, 3-Amino-1-cyclohexylaminopropan, 3-Amino-1-Methylaminobutan, weiterhin Aminoalkohole, wie N-Aminoethylethanolamin, Ethanolamin, 3-Aminopropanol, Neopentanolamin und besonders bevorzugt Diethanolamin.

Der Bestandteil c) der Polyurethanharnstoffe kann bei deren Herstellung als Kettenverlängerer eingesetzt werden.

Die Menge an Bestandteil c) bei der Herstellung der Polyurethallharnstoffe beträgt vorzugsweise 0,1 bis 1,5 mol, besonders bevorzugt 0,2 bis 1,3 mol, insbesondere 0,3 bis 1,2 mol, jeweils bezogen auf die Menge der Verbindungen der Komponente a).

In einer weiteren Ausführungsform umfassen die Polyurethanharnstoffe zusätzliche Einheiten, welche auf mindestens ein weiteres Polyol d) als Aufbaukomponente zurückgehen.

Die zum Aufbau der Polyurethanharnstoffe eingesetzten weiteren niedermolekularen Polyole d) bewirken in der Regel eine Versteifung und/oder eine Verzweigung der Polymerkette. Das Molekulargewicht beträgt vorzugsweise 62 bis 500 g/mol, besonders bevorzugt 62 bis 400 g/mol, insbesondere 62 bis 200 g/mol.

Geeignete Polyole können aliphatische, alicyclische oder aromatische Gruppen enthalten. Genannt seien hier beispielsweise die niedermolekularen Polyole mit bis zu etwa 20 Kohlenstoffatomen je Molekül, wie z. B. Ethyenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), sowie Trimethylolpropan, Glycerin oder Pentaerythrit und Mischungen dieser und gegebenenfalls auch weiterer niedermolekularer Polyole. Auch Esterdiole wie z.B. α-Hydroxybutyl-ε-hydroxy-capronsäureester, ω-Hydroxyhexyl-γ-hydroxybuttersäure-ester, Adipinsäure-(ß-hydroxyethyl)ester oder Terephthalsäure-bis(ß-hydroxyethyl)-ester können verwendet werden.

Die Menge an Bestandteil d) bei der Herstellung der Polyurethanharnstoffe beträgt vorzugsweise 0,05 bis 1,0 mol, besonders bevorzugt 0,05 bis 0,5 mol, insbesondere 0,1 bis 0,5 mol, jeweils bezogen auf die Menge der Verbindungen der Polycarbonatpolyolkomponente a).

Die Umsetzung der isocyanathaltigen Komponente b) mit den hydroxy- oder aminfunktionellen Verbindungen a), c) und gegebenenfalls d) erfolgt üblicherweise unter Einhaltung eines leichten NCO-Überschusses gegenüber den reaktiven Hydroxy- oder Aminverbindungen. Am Endpunkt der Reaktion durch Erreichen einer Zielviskosität verbleiben immer noch Reste an aktivem Isocyanat. Diese Reste müssen blockiert werden, damit nicht eine Reaktion mit großen Polymerketten stattfindet. Eine solche Reaktion führt zur dreidimensionalen Vernetzung und Vergelung des Ansatzes. Die Verarbeitung einer solchen Lösung ist nicht mehr möglich. Üblicherweise enthalten die Ansätze hohe Mengen an Alkoholen. Diese Alkohole blockieren innerhalb von mehreren Stunden Stehen oder Rühren des Ansatzes bei Raumtemperatur die noch verbliebenen Isocyanatgruppen.

Wurde bei der Herstellung der Polyurethanharnstoffe der Restisocyanatgehalt blockiert, weisen diese auch Monomere als Aufbaukomponenten e) auf, die sich jeweils an den Kettenenden befinden und diese abschließen.

Diese Aufbaukomponenten e) leiten sich zum einen von monofunktionellen, mit NCO-Gruppen reaktiven Verbindungen, wie Monoaminen, insbesondere von mono-sekundären Aminen oder Monoalkoholen ab. Genannt seien hier beispielsweise Ethanol, n-Butanol, Ethylenglykol-monobutylether, 2-Ethylhexanol, 1-Octanol, 1-Dodecanol, 1-Hexadecanol, Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin und geeignete substituierte Derivate davon.

Da die Aufbaukomponenten e) im Wesentlichen in den Polyurethanharnstoffen dazu verwendet werden, den NCO-Überschuss zu vernichten, hängt die erforderliche Menge im Wesentlichen von der Menge des NCO-Überschusses ab und kann nicht allgemein spezifiziert werden.

Bevorzugt wird während der Synthese auf die Aufbaukomponente e) verzichtet. Noch nicht umgesetztes Isocyanat wird dabei vorzugsweise durch die in sehr großen Konzentrationen enthaltenen Lösungsmittelalkohole zu terminalen Urethanen umgesetzt.

Zur Herstellung der erfindungsgemäßen Polyurethanlösungen werden die Polycarbonatpolyolkomponente a), das Polyisocyanat b) und gegebenenfalls das Polyol d) in der Schmelze oder in Lösung miteinander umgesetzt, bis alle Hydroxylgruppen verbraucht sind.

Die dabei verwendete Stöchiometrie zwischen den einzelnen an der Umsetzung beteiligten Komponenten ergibt sich aus den zuvor erwähnten Mengenverhältnissen.

Die Umsetzung erfolgt bei einer Temperatur von vorzugsweise 60 bis 110 °C, besonders bevorzugt 75 bis 110 °C, insbesondere 90 bis 110 °C, wobei Temperaturen um 110 °C aufgrund der Geschwindigkeit der Umsetzung bevorzugt sind. Höhere Temperaturen können ebenfalls angewendet werden, allerdings besteht dann im Einzelfall und in Abhängigkeit der einzelnen verwendeten Komponenten das Risiko, dass Zersetzungsprozesse und Verfärbungen in dem entstehenden Polymer auftreten.

Bei dem Prepolymer aus Isocyanat und allen Hydroxylgruppen aufweisenden Komponenten ist die Umsetzung in Schmelze bevorzugt, allerdings besteht die Gefahr, dass es zu hohen Viskositäten der ausreagierten Gemische kommt. In diesen Fällen empfiehlt es sich auch, Lösungsmittel hinzuzugegeben. Es sollte aber möglichst nicht mehr als ungefähr 50 Gew.-% Lösungsmittel enthalten sein, da andernfalls die Verdünnung die Reaktionsgeschwindigkeit deutlich verlangsamt.

Bei der Umsetzung von Isocyanat- und Hydroxylgruppen-aufweisenden Komponenten kann die Reaktion in der Schmelze in einem Zeitraum von 1 Stunde bis 24 Stunden erfolgen. Geringe Zugaben von Lösungsmittelmengen führen zu einer Verlangsamung, wobei die Umsetzungszeiträume jedoch in den gleichen Zeiträumen liegen.

Die Reihenfolge der Zugabe bzw. Umsetzung der einzelnen Komponenten kann von der zuvor angegebenen Reihenfolge abweichen. Dies kann insbesondere dann von Vorteil sein, wenn die mechanischen Eigenschaften der aus den Polyurethanharnstoff herstellbaren Beschichtungen verändert werden sollen. Wenn man beispielsweise alle Hydroxylgruppen aufweisenden Komponenten gleichzeitig umsetzt, entsteht ein Gemisch aus Hart- und Weichsegmenten. Wenn man beispielsweise das niedermolekulare Polyol nach der Polycarbonatpolyolkomponente zugibt, erhält man definierte Blöcke, was andere Eigenschaften der resultierenden Beschichtungen mit sich bringen kann. Die vorliegende Erfindung ist somit nicht auf eine bestimmte Reihenfolge der Zugabe bzw. Umsetzung der einzelnen Komponenten beschränkt.

Nach diesen Umsetzungsschritten kann weiteres Lösungsmittel zugesetzt und gegebenenfalls gelöstes Kettenverlängerungsdiamin bzw. gelöster Kettenverlängerungsaminoalkohol (Komponente (c)) zugegeben werden.

Die weitere Zugabe des Lösungsmittels erfolgt vorzugsweise schrittweise, um die Reaktion nicht unnötig zu verlangsamen, was bei einer vollständigen Zugabe der Lösungsmittelmenge beispielsweise am Anfang der Umsetzung passieren würde. Ferner ist man bei einem hohen Gehalt an Lösungsmittel zum Beginn der Reaktion an eine im Verhältnis niedrige Temperatur gebunden, welche von der Art des Lösungsmittels zumindest mitbestimmt wird. Auch dieses führt zu einer Verlangsamung der Reaktion.

Nach Erreichen der Zielviskosität können die noch verbleibenden Reste an NCO durch ein monofunktionelles aliphatisches Amin blockiert werden. Bevorzugt blockiert man die noch verbliebenen Isocyanatgruppen durch Umsetzung mit den im Lösungsmittelgemisch enthaltenen Alkoholen.

Die erfindungsgemäßen Polyurethanharnstoffe können ferner für den jeweils angestrebten Einsatzzweck übliche Bestandteile und Additive enthalten.

Ein Beispiel hierfür sind pharmakologische Wirkstoffe und Additive, die die Freisetzung von pharmakologischen Wirkstoffen fördern ("drug-eluting-Additive"). Eine bevorzugte Ausführungsform sieht vor, dass der Polyurethanharnstoff pharmakologische Wirkstoffe enthält.

Pharmakologische Wirkstoffe, die in Beschichtungen auf medizinischen Artikeln verwendet werden können, sind beispielsweise thromboresistente Mittel, antibiotische Mittel, Antitumormittel, Wachtumshormone, Antivirusmittel, antiangiogene Mittel, angiogene Mittel, antimitotische Mittel, entzündungshemmende Mittel, Zellzyklus-regulierende Mittel, genetische Mittel, Hormone, sowie deren Homologe, Derivate, Fragmente, pharmazeutische Salze und Kombinationen davon.

Spezifische Beispiele pharmakologischer Wirkstoffe schließen somit thromboresistente (nichtthrombogene) Mittel bzw. andere Mittel zur Unterdrückung einer akuten Thrombose, Stenose oder späten Re-Stenose der Arterien ein. Dies sind beispielsweise Heparin, Streptokinase, Urokinase, Gewebe-Plasminogen-Aktivator, Anti-Thromboxan-B₂-Mittel; Anti-B-Thromoboglobulin, Prostaglandin-E, Aspirin, Dipyridimol, Anti-Thromboxan-A₂-Mittel, muriner monoklonaler Antikörper 7E3, Triazolopyrimidin, Ciprosten, Hirudin, Ticlopidin, Nicorandil usw.

Ein Wachstumsfaktor kann als ebenfalls als ein pharmakologischer Wirkstoff benutzt werden, um unterintimale fibromüskulare Hyperplasie an der arteriellen Stenosestelle zu unterdrücken, bzw. es kann jeder beliebige andere Hemmstoff des Zellwachstums an der Stenosestelle eingesetzt werden.

Der pharmakologische Wirkstoff kann auch aus einem Vasodilatator bestehen, um Vasospasmus entgegen zu wirken. Dabei kann es sich zum Beispiel ein Antispasmusmittel wie Papaverin handeln. Der pharmakologische Wirkstoff kann ein vasoaktives Mittel an sich wie Calciumantagonisten oder α- und β-adrenergische Agonisten oder Antagonisten sein. Zusätzlich kann der pharmakologische Wirkstoff ein biologisches Haftmittel wie Cyanoacrylat in medizinischer Qualität oder Fibrin sein.

Der pharmakologische Wirkstoff kann ferner ein antineoplastisches Mittel wie 5-Fluorouracil, vorzugsweise mit einem kontrollierenden freisetzenden Träger für das Mittel, sein, z.B. für die Anwendung eines fortdauernden kontrollierten freisetzenden antineoplastischen Mittels an einer Tumorstelle.

Der pharmakologische Wirkstoff kann ein Antibiotikum, vorzugsweise in Kombination mit einem kontrollierenden freisetzenden Träger für die fortdauernde Freisetzung aus der Beschichtung eines medizinischen Artikels an einen lokalisierten Infektionsherd innerhalb des Körpers, sein. Ähnlich kann der pharmakologische Wirkstoff Steroide für den Zweck des Unterdrückens einer Entzündung in lokalisiertem Gewebe oder aus anderen Gründen enthalten.

Spezifische Beispiele geeigneter pharmakologischer Wirkstoffe umfassen:
(a) Heparin, Heparinsulfat, Hirudin, Hyaluronsäure, Chondroitinsulfat, Dermatansulfat, Keratansulfat, lytische Mittel, einschließlich Urokinase und Streptokinase, deren Homologe, Analoge, Fragmente, Derivate und pharmazeutische Salze davon;
(b) antibiotischen Mitteln wie Penicilline, Cephalosporine, Vacomycine, Aminoglycoside, Quinolone, Polymyxine, Erythromycine; Tetracycline, Chloramphenicole, Clindamycine, Lincomycine, Sulfonamide, deren Homologe, Analoge, Derivate, pharmazeutische Salze und Mischungen davon;
(c) Paclitaxel, Docetaxel, Immunsuppressiva wie Sirolimus oder dem Sirolimus verwandte Limus-Derivate wie beispielsweise Everolimus, Biolimus A9, Tacrolimus oder Zotarolimus, Alkylierungsmittel einschließlich Mechlorethamin, Chlorambucil, Cyclophosphamid, Melphalan und Ifosfamid; Antimetaboliten einschließlich Methotrexat, 6-Mercaptopurin, 5-Fluorouracil und Cytarabin; Pflanzenalkoide einschließlich Vinblastin; Vincristin und Etoposid; Antibiotika einschließlich Doxorubicin, Daunomycin, Bleomycin und Mitomycin; Nitrosurea einschließlich Carmustin und Lomustin; anorganische Ionen einschließlich Cisplatin; biologische Reaktionsmodifikatoren einschließlich Interferon; angiostatinische Mittel und endostatinische Mittel; Enzyme einschließlich Asparaginase; und Hormone einschließlich Tamoxifen und Flutamid, deren Homologe, Analoge, Fragmente, Derivate, pharmazeutische Salze und Mischungen davon;
(d) antivirale Mittel wie Amantadin, Rimantadin, Rabavirin, Idoxuridin, Vidarabin, Trifluridin, Acyclovir, Ganciclorir, Zidovudin, Phosphonoformate, Interferone, deren Homologe, Analoge, Fragmente, Derivate, pharmazeutische Salze und Mischungen davon; und
(e) entzündungshemmende Mittel wie beispielsweise Ibuprofen, Dexamethason oder Methylprednisolon.

Gegenstand der Erfindung ist weiterhin ein Substrat mit einer darauf aufgebrachten Grundbeschichtung aus einem erfindungsgemäßen Polyurethanharnstoff.

Vorzugsweise kann auf der Grundbeschichtung eine Deckbeschichtung aus einem erfindungsgemäßen Polyurethanharnstoff aufgebracht sein, die sich in ihren chemischen und / oder physikalischen Eigenschaften von der Grundbeschichtung unterscheidet.

Insbesondere kann die Grundbeschichtung einen pharmakologischen Wirkstoff enthalten.

Die Deckbeschichtung kann eine deutlich niedrigere Konzentration an Wirkstoff als die Grundbeschichtung enthalten, d.h. beispielsweise weniger als 10 % der Menge an Wirkstoff, der pro Volumeneinheit in der Grundbeschichtung enthalten ist. Besonders bevorzugt ist, wenn die Deckbeschichtung wirkstofffrei oder nahezu wirkstofffrei ist. Durch das Vorhandensein der Deckbeschichtung wird die Abgabe des Wirkstoffs aus der Grundbeschichtung weiter verlangsamt.

Eine besonders bevorzugte Ausführungsform des erfindungemäßen Substrat sieht vor, dass die Grundbeschichtung eine Schichtdicke von 5 bis 20 µm und / oder die Deckbeschichtung eine Schichtdicke von 0,5 bis 10 µm hat.

Bei dem Substrat kann es sich insbesondere um einen medizinischen Artikel handeln.

Die Bezeichnung "medizinischer Artikel" ist im Rahmen der vorliegenden Erfindung breit zu verstehen. Geeignete, nicht einschränkende Beispiele für medizinische Artikel sind Kontaktlinsen; Kanülen; Katheter, zum Beispiel urologische Katheter wie Blasenkatheter oder Harnleiterkatheter; zentralvenöse Katheter; venöse Katheter oder Einlass- bzw. Auslass-Katheter; Dilationsballons; Katheter für die Angioplastie und die Biopsie; Katheter, die für das Einbringen eines Stents, eines Propf- oder eines Kavafilters verwendet werden; Ballonkatheter oder andere dehnbare medizinische Geräte; Endoskopiegeräte; Larnygoskopiegeräte; Trachealgeräte wie Endotrachealschläuche, Atemgeräte und andere Trachealabsauggeräte; bronchoalveolare Spülungskatheter; Katheter, die bei der Koronarangioplastie verwendet werden; Führungsstäbe, Einführer und Ähnliches; Gefäßpfropfen; Schrittmacherteile; Cochleaimplantate; Zahnimplantatschläuche für die Nahrungszufuhr, Dränageschläuche; und Führungsdrähte.

Darüber hinaus können die erfindungsgemäßen Polyurethanharnstoffe zur Herstellung von Beschichtungen, zum Beispiel für Handschuhe, Stents und andere Implantate; extrakorporale Blutschläuche; Membrane, zum Beispiel für die Dialyse; Blutfilter; Geräte für die Kreislaufunterstützung; Verbandsmaterial für die Wundpflege; Harnbeutel und Stomabeutel verwendet werden. Eingeschlossen sind auch Implantate, die ein medizinisch wirksames Mittel enthalten, wie medizinisch wirksame Mittel für Stents oder für Ballonoberflächen oder für Kontrazeptiva.

Ganz besonders bevorzugt handelt es sich bei dem medizinischen Artikel um einen implantierbar Artikel und insbesondere um einen Stent.

Noch ein weiterer Gegenstand der Erfindung ist ein Schichtaufbau umfassend wenigstens eine wirkstoffhaltige Schicht aus einem erfindungsgemäßen Polyurethanharnstoff und wenigstens eine wirkstofffreie Schicht aus einem erfindungsgemäßen Polyurethanharnstoff.

Ein Verfahren zum Beschichten eines Substrats, bei dem auf das Substrat wenigstens eine Schicht aus einem erfindungsgemäßen Polyurethanharnstoff aufgebracht wird, ist ebenfalls Gegenstand der Erfindung.

Vorzugsweise wird dabei auf das Substrat eine Grundbeschichtung aus einem wirkstoffhaltigen Polyurethanharnstoff und auf die Grundbeschichtung eine Deckbeschichtung aus einem wirkstofffreien Polyurethanharnstoff aufgebracht.

### Beispiele:

Die Erfindung wird im Folgenden anhand von Beispielen im Detail erläutert.

### Methoden:

Die Ermittlung des NCO-Gehaltes der in den Beispielen und Vergleichsbeispielen beschriebenen Harze erfolgte durch Titration gemäß DIN EN ISO 11909.

Die Bestimmung der Festkörpergehalte erfolgte nach DIN-EN ISO 3251. Hierzu wurden 1-1,5 g Polyurethanharnstofflösung bei 50 °C ca. 17 h im Vakuumtrockenschrank bis zur Gewichtskonstanz getrocknet.

Die in % angegebenen Mengenangaben verstehen sich, wenn nicht anders vermerkt, als Gew.-% und beziehen sich auf die erhaltene organisch Polyurethanharnstofflösung.

Die OH-Zahlen wurden nach DIN 53240 bestimmt.

Viskositätsmessungen wurden mit dem Physics MCR 51 Rheometer der Firma Anton Paar GmbH, Ostfildern, Deutschland, durchgeführt.

### Verwendete Substanzen und Abkürzungen:

| | |
|---|---|
| Desmophen C2200: | Polycarbonatpolyol, OH-Zahl 56 mg KOH/g, zahlenmittleres Molekulargewicht 2000 g/mol (Bayer MaterialScience AG, Leverkusen, DE) |
| Polyether LB 25: | monofunktioneller Polyether auf Ethylenoxid-/Propylenoxidbasis zahlenmittleres Molekulargewicht 2250 g/mol, OH-Zahl 25 mg KOH/g (Bayer MaterialScience AG, Leverkusen, DE) |
| TCD-Alkohol DM | 3(4),8(9)-Bis(hydroxymethyl)-tricyclo(5.2.1.0/2.6)decan/ Tricyclodecandimethanol, Celanese Corp., Dallas, USA |
| Sirolimus: | Sirolimus (aus *Streptomyces hygroscopicus*; CAS: 53123-88-9; Poli Industria Chimica S.p.A; Rozzano, Italien) |
| Verwendete Stents: | CoCr-Stents für koronare Indikation aus der Serie des ProKinetik-Stents der Biotronik (Berlin, Deutschland) ohne die serienmäßige Si-Carbid-Beschichtung, 18 mm lang mit 60 µm Wandstärke und einer Gesamtoberfläche von 76,56 mm² |

### Beispiel 1:

### Herstellung eines cycloaliphatischen Polycarbonatdiols auf Basis TCD-Alkohol DM mit einem zahlenmittleren Molekulargewicht von 1.300 g/mol

In einem 16 1 Druckreaktor mit Destillationsaufsatz, Rührer und Vorlage wurden 5436 g TCD Alkohol DM mit 1,2 g Yttrium(III)acetylacetonat sowie 3810 g Dimethylcarbonat bei 80 °C vorgelegt. Anschließend wurde unter Stickstoffatmosphäre das Reaktionsgemisch in 2 h auf 135 °C aufgeheizt und dort unter Rühren 24 h gehalten, wobei der Druck auf 6,3 bar (absolut) anstieg. Danach wurde auf 60 °C abgekühlt und belüftet. Dann wurde das Spaltprodukt Methanol im Gemisch mit Dimethylcarbonat per Destillation entfernt, wobei die Temperatur schrittweise auf 150 °C erhöht wurde. Es wurde dann noch 4 Stunden bei 150 °C gerührt, anschließend auf 180 °C aufgeheizt und dann noch 4 h bei 180 °C gerührt. Dann wurde die Temperatur auf 90 °C reduziert und ein Stickstoffstrom (5 l/h) durch das Reaktionsgemisch hindurchgeleitet, während der Druck auf 20 mbar abgesenkt wurde. Danach wurde die Temperatur binnen 4 h auf 180 °C erhöht und dort 6 h gehalten. Dabei erfolgte die weitere Entfernung von Methanol im Gemisch mit Dimethylcarbonat aus dem Reaktionsgemisch.

Nach Belüftung und Abkühlung des Reaktionsansatzes auf Raumtemperatur, wurde ein gelbliches, festes Polycarbonatdiol mit folgenden Kennzahlen erhalten:
Mₙ = 1.290 g/mol; OH-Zahl = 87 mg KOH/g;

### Beispiel 2: (erfindungsgemäß)

97,8 g Desmophen C 2200, 63,6 g Polycarbonatdiol des Beispiels 1 und 47,8 g 4,4'-Bis(isocyanatocyclohexyl)methan (H₁₂MDI) wurden bei 110 °C 4 Stunden bis zu einem konstanten NCO-Gehalt von 3,3 % umgesetzt. Man ließ abkühlen und verdünnte mit 335 g Toluol und 185 g iso-Propanol. Bei Raumtemperatur wurden eine Lösung von 12,6 g Isophorondiamin in 92,0 g 1-Methoxypropanol-2 zugegeben. Nach beendetem Aufbau des Molgewichtes und Erreichen des gewünschten Viskositätsbereiches rührte man weitere 15 Stunden bei Raumtemperatur, um den restlichen Isocyanatgehalt mit iso-Propanol zu blockieren. Man erhielt 833,8 g einer 27,0%igen Polyurethanharnstofflösung in Toluol/iso-Propanol/1-Methoxypropanol-2 mit einer Viskosität von 46400 mPas bei 23 °C.

### Beispiel 3: (Vergleich)

Dieses Beispiel beschreibt die Synthese einer hydrophoben Beschichtung ohne Zusatz des Polycarbonatdiols des Beispiels 1.

195,4 g Desmophen C 2200 und 47,8 g 4,4'-Bis(isocyanatocyclohexyl)methan (H₁₂MDI) wurden bei 110 °C 17 Stunden bis zu einem konstanten NCO-Gehalt von 2,8 % umgesetzt. Man ließ abkühlen und verdünnte mit 350 g Toluol und 200 g iso-Propanol. Bei Raumtemperatur wurden eine Lösung von 11,5 g Isophorondiamin in 85,0 g 1-Methoxypropanol-2 zugegeben. Nach beendetem Aufbau des Molgewichtes und Erreichen des gewünschten Viskositätsbereiches rührte man weitere 20 Stunden bei Raumtemperatur, um den restlichen Isocyanatgehalt mit iso-Propanol zu blockieren. Man erhielt 889,7 g einer 29,3%igen Polyurethanharnstofflösung in Toluol/iso-Propanol/1-Methoxypropanol-2 mit einer Viskosität von 24600 mPas bei 23 °C.

### Beispiel 4: (Vergleich)

Dieses Beispiel beschreibt die Synthese einer hydrophilen Beschichtung ohne Zusatz des Polycarbonatdiols des Beispiels 1.

195,4 g Desmophen C 2200, 40,0 g LB 25 und 47,8 g 4,4'-Bis(isocyanatocyclohexyl)methan (H₁₂MDI) wurden 19 h bei 110 °C bis zu einem konstanten NCO-Gehalt von 2,2 % umgesetzt. Man ließ abkühlen und verdünnte mit 350 g Toluol und 200 g iso-Propanol. Bei Raumtemperatur wurden eine Lösung von 12,0 g Isophorondiamin in 100 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 945,2 g einer 31,6%igen Polyurethanharnstofflösung in Toluol/iso-Propanol/1-Methoxypropanol-2 mit einer Viskosität von 19300 mPas bei 23 °C.

### Beispiel 5: (Vergleich)

Dieses Beispiel beschreibt die Synthese einer hydrophilen Beschichtung unter Zusatz des Polycarbonatdiols des Beispiel 1.

97,8 g Desmophen C 2200, 63,6 g Polycarbonatdiol des Beispiels 1, 30,0 g LB 25 und 47,8 g 4,4'-Bis(isocyanatocyclohexyl)methan (H₁₂MDI) wurden bei 110 °C 2 Stunden bis zu einem konstanten NCO-Gehalt von 2,6 % umgesetzt. Man ließ abkühlen und verdünnte mit 335 g Toluol und 185 g iso-Propanol. Bei Raumtemperatur wurden eine Lösung von 12,0 g Isophorondiamin in 94,0 g 1-Methoxypropanol-2 zugegeben. Nach beendetem Aufbau des Molgewichtes und Erreichen des gewünschten Viskositätsbereiches rührte man weitere 15 Stunden, um den restlichen Isocyanatgehalt mit iso-Propanol zu blockieren. Man erhielt 865,2 g einer 31,0%igen Polyurethanharnstofflösung in Toluol/iso-Propanol/1-Methoxypropanol-2 mit einer Viskosität von 33300 mPas bei 23 °C.

### Beispiel 6: (Vergleich)

586,2 g Desmophen C 2200, 21,6 g Butandiol-1,4 und 141,3 g 4,4'-Bis(isocyanatocyclohexyl)methan (H₁₂MDI) werden 140 min bei 150 °C umgesetzt. Das warme Reaktionsgemisch wird in eine auf 80 °C vorgewärmte Schale ausgegossen und 2 h bei 90 °C im Trockenschrank gelagert. Nach Abkühlen erhält man ein festes Produkt, welches zur Beschichtung von Stents in Lösungsmittel aufgenommen werden muss. Hierzu werden 30 g des erhaltenen festen Produktes in 70 g Toluol/Isopropanol-Gemisch (64 Gew% Toluol, 36 Gew% Isopropanol) eingetragen und 4 h bei Raumtemperatur gerührt. Man erhält eine klare Lösung ohne ungelöste Bestandteile mit einer Viskosität von 640 mPas bei 23 °C.

### Beispiel 7: Herstellung der Beschichtungslösung für die Stentbeschichtung

Die Polyurethan-Stammlösung der Beispiels 2 mit einem Polymeranteil von 27,0 Gew% wurde mit einem Gemisch aus 54 % Toluol und 46 % 2-Propanol in einem Verhältnis von 1:80 verdünnt, so dass ein Polymeranteil von ∼0,34Gew% erreicht wurde. Zu der verdünnten Lösung wurden 15 Gew% des Wirkstoffs (Sirolimus bzw. Paclitaxel) bezogen auf die Polymermasse als methanolische Lösung (∼5 mg/mL) hinzugegeben. Dazu wurden für eine Beschichtungslösung 0,5 g der Polyurethan-Stammlösung in einen Erlenmeyerkolben eingewogen und 40 g des Toluol/2-Propanol-Gemischs zur Verdünnung unter Rühren hinzugefügt. Anschließend wurden 20 mg Paclitaxel bzw. Sirolimus in 4 mL Methanol gelöst und ebenfalls unter Rühren zugegeben.

In gleicher Weise wurden die Polyurethan-Stammlösungen der Beispiele 3, 4, 5 und 6 verdünnt und ebenfalls mit 15 Gew% des Wirkstoffes, bezogen auf die Polymermasse, versetzt.

### Beispiel 8: Allgemeines Verfahren für die Stentbeschichtung

### a) Einschichtsystem

Vor der Beschichtung wurden die Stents mit Chloroform im Ultraschallbad gereinigt. Anschließend wurden die gereinigten Stents lichtmikroskopisch begutachtet und gegebenenfalls erneut gereinigt. Die Ausgangsmasse der unbeschichteten Stents wurde mit Hilfe einer Ultramikrowaage bestimmt.

Die Stents wurden mit Hilfe einer Sprühbeschichtungsanlage beschichtet. Die Beschichtungstechnik beruht darauf, dass eine Beschichtungslösung gemäß Beispiel 7 durch Druckluft in einer Düse mit einem Sprühdruck von 0,3 - 0,5 bar zerstäubt wird. Der Innendurchmesser der verwendeten Sprühdüse kann zwischen 0,1 und 3 mm betragen. Der zu beschichtende Stent befindet sich dabei in einem Halter, der im Sprühstrahl positioniert ist und den Stent um seine Längsachse dreht. Der Abstand zwischen Stent und Düse kann zwischen 10 und 100 mm betragen. Der Fortschritt des Beschichtungsprozesses wird dabei durch Wägung der Stents und Differenzbildung gegen die Ausgangsmassen bestimmt. Nach erfolgter vollständiger Beschichtung werden die Stents bei 40 °C zwischen 12 h und 24 h im Vakuumtrockenschrank bei einem Druck von etwa 10 mbar getrocknet.

### b) Mehrschichtsystem

Eine erste Grundschicht besteht aus der im Beispiel 7 beschriebenen verdünnten Polymerstammlösungen (hergestellt aus den Polyurethanlösungen der Beispiele 2-6), denen die in Beispiel 7 genannten Mengen an Sirolimus zugesetzt wurden. Stents wurden, wie im vorherigen Absatz beschrieben, mit diesen wirkstoffhaltigen Polyurethanlösungen beschichtet und wie angegeben getrocknet. Auf die getrocknete, wirkstoffhaltige Polyurethanbeschichtung wurden dann in einem zweiten Arbeitsgang die reinen verdünnten Polyurethanlösungen des Beispiels 7 ohne Anteil an Sirolimus als Deckschicht aufgetragen und ebenfalls wie angegeben getrocknet. Als Deckschicht wurde jeweils dieselbe Polyurethanlösung genommen, die auch als wirkstoffhaltige Matrix verwendet wurde.

### c) Schichtdickenbestimmung

Mittels konfokalem Lasermikroskop (Olympus LEXT OLS 300) wurden an in der beschriebenen Weise beschichteten Stents Messungen zur Bestimmung der Schichtdicke der aufgetragenen Polymer-Wirkstoffschichten durchgeführt. Bei den gewählten Grundbeschichtungsmassen von 1000 - 1100 µg findet man an verschiedenen Messpunkten an einem Stent Schichtdicken von 6 µm bis 14 µm. Eine Deckschicht mit einer Beschichtungsmasse von 100 µg ergibt eine Schichtdicke von 1,2 µm bis 1,4 µm. Deckschichten mit einer Beschichtungsmasse von 700 µg ergeben Schichtdicken von 8 µm bis 9 µm.

### Beispiel 9: Allgemeines Verfahren für die Untersuchung der Medikamentenfreisetzung

Die gemäß Beispiel 8 beschichteten Stents wurden manuell auf einen Ballonkatheter (Ballon-katheter vom Stentsystem Lekton 3.0/20, Quelle Biotronik, Berlin, Deutschland) gecrimpt. Der gecrimpte Stent wurde jeweils in ein mit einem Schraubdeckel verschließbares Glasvial, in dem 2 mL einer auf 37 °C temperierten 0,9%igen NaCl_{[aq]}-Lösung (enthält noch 0,05 Gew% nichtionisches Detergens Brij 35 sowie 3 mg/L Antioxidans BHT (Butylhydroxytoluol)) vorgelegt wurden, eingetaucht und dann mit Hilfe einer Handpumpe (Guidant, Boston Scientific) bei einem Druck von 10 bar dilatiert. Das Glasvial wurde verschlossen und mit einem Schüttler (IKA MS 3 basic) bei 37 °C langsam geschüttelt. Nach einer vorher definierten Zeit wurde der Stent aus dem Elutionsmedium entnommen und auf einem Zellstofftuch getrocknet. Anschließend wurde der Stent erneut in ein Vial mit 2 mL Elutionsmedium gegeben und bei 37 °C geschüttelt.

Die Bestimmung der freigesetzten Wirkstoffmenge wurde mit Hilfe einer HPLC-Apparatur (Knauer Berlin; UV-Delektor K-2501; HPLC-Punpe K-1001; Solvent-Organizer K-1500; Smartline Autosampler 3800; Jet Stream Oven, Säule Eurospher-100, C18, 120 x 4 mm) durchgeführt. Bei einem Fluss von 1 mL/min wurde für Sirolimus eine Mischung aus Reinstwasser und Acetonitril (35/65; v/v) als Laufmittel genutzt, für Paclitaxel dagegen wurde eine Mischung aus Acetonitril und einer wässrigen KH₂PO₄-Lösung (pH = 3,5) (50/50, v/v) genutzt. Der UV-Detektor war während der Messung auf 254 nm Messwellenlänge eingestellt.

### Beispiel 10: Ergebnisse der Freisetzungsstudien

Ziel der Erfindung ist die Entwicklung einer Stentbeschichtung, die über mehrere Wochen kontinuierlich den Wirkstoff Sirolimus freisetzt. Hierzu wurden gemäß der Vorschriften der Beispiele 7-9 Stents hergestellt, die neben einer wirkstoffhaltigen Polyurethanschicht als Grundbeschichtung zusätzlich noch steigende Mengen an wirkstofffreier Polyurethandeckschicht enthalten. Die im Folgenden aufgeführten Tabellen enthalten die Freisetzungsraten von Sirolimus aus wirkstoffhaltigen Polyurethanbeschichtungen ohne wirkstofffreie Deckbeschichtung sowie mit steigenden Massen an wirkstofffreien Deckschichten.

Für jede Beschichtung werden zwei Tabellen und Grafiken gezeigt: die Freisetzung der absoluten Menge an Sirolimus sowie die Freisetzung als Prozentanteil vom eingesetzten Sirolimus. Die Werte beinhalten die zum jeweiligen Zeitpunkt kumulativ freigesetzten Mengen.

### 1. Stents mit Beschichtung aus Polyurethan des Beispiels 2 (erfinderisch)

| | Grundbeschichtung (µg) | Sirolimus (µg) | Deckbeschichtung (µg) |
|---|---|---|---|
| Stent 1 | 1058 | 158,7 | 0 |
| Stent 2 | 1081 | 162,15 | 108 |
| Stent 3 | 1063 | 159,45 | 311 |
| Stent 4 | 1113 | 166,95 | 504 |
| Stent 5 | 1048 | 157,2 | 714 |

| Zeit (h) | Stent 1 Summe abs. (µg) | Zeit (h) | Stent 2 Summe abs. (µg) | Zeit (h) | Stent 3 Summe abs. µg) | Zeit (h) | Stent 4 Summe abs.µg) | Zeit (h) | Stent 5 Summe abs. (µg) |
|---|---|---|---|---|---|---|---|---|---|
| | | | | | | | | | |
| 0 | 0 | 0 | 0 | 0 | 0 | 0 | 0 | 0 | 0 |
| 0,33 | 9,836 | 0,33 | 1,836 | 0,3 3 | 0,704 | 0,3 3 | 0,204 | 0,3 3 | 0,152 |
| 0,67 | 14,324 | 0,67 | 2,508 | 0,6 7 | 1,024 | 0,6 7 | 0,32 | 0,6 7 | 0,268 |
| 1 | 17,924 | 1 | 3,036 | 1 | 1,208 | 1 | 0,476 | 1 | 0,372 |
| 1,5 | 22,64 | 1,5 | 3,744 | 1,5 | 1,572 | 1,5 | 0,648 | 1,5 | 0,52 |
| 2 | 26,016 | 2 | 4,644 | 2 | 1,992 | 2 | 0,86 | 2 | 0,664 |
| 3 | 32,132 | 3 | 5,948 | 3 | 2,632 | 3 | 1,184 | 3 | 0,876 |
| 4 | 37,132 | 4 | 7,12 | 4 | 3,248 | 4 | 1,512 | 4 | 1,14 |
| 5 | 41,164 | 5 | 8,252 | 5 | 3,832 | 5 | 1,84 | 5 | 1,388 |
| 6 | 44,652 | 6 | 9,312 | 6 | 4,396 | 6 | 2,196 | 6 | 1,608 |
| 7 | 47,864 | 7 | 10,324 | 7 | 4,98 | 7 | 2,496 | 7 | 1,848 |
| 9 | 53,184 | 9 | 12,18 | 9 | 6,056 | 9 | 3,128 | 9 | 2,32 |
| 11 | 57,876 | 11 | 14,024 | 11 | 7,148 | 11 | 3,756 | 11 | 2,82 |
| 13 | 61,98 | 13 | 15,68 | 13 | 8,104 | 13 | 4,384 | 13 | 3,256 |
| 16 | 66,528 | 16 | 18,204 | 16 | 9,52 | 16 | 5,368 | 16 | 3,904 |
| 19 | 70,744 | 19 | 20,488 | 19 | 10,852 | 19 | 6,32 | 19 | 4,612 |
| 22 | 74,212 | 22 | 22,816 | 22 | 12,304 | 22 | 7,28 | 22 | 5,364 |
| 26 | 78,256 | 27 | 26,288 | 27 | 14,184 | 27 | 8,628 | 27 | 6,376 |
| 32 | 82,144 | 33 | 29,648 | 33 | 16,164 | 33 | 10,124 | 33 | 7,584 |
| 39 | 85,716 | 40 | 33,696 | 40 | 18,624 | 40 | 12,036 | 40 | 8,972 |
| 47 | 89,468 | 46 | 37,124 | 46 | 20,872 | 46 | 13,784 | 46 | 10,336 |
| 54 | 92,248 | 53 | 40,308 | 53 | 23,196 | 53 | 15,492 | 53 | 11,776 |
| 61 | 94,728 | 60 | 43,78 | 60 | 25,516 | 60 | 17,404 | 60 | 13,152 |
| 68 | 97,004 | 67 | 46,62 | 67 | 27,424 | 67 | 18,976 | 67 | 14,356 |
| 75 | 98,972 | 74 | 49,52 | 74 | 29,484 | 74 | 20,36 | 74 | 15,764 |
| 82 | 100,88 | 81 | 52,5 | 81 | 31,56 | 81 | 21,892 | 81 | 17,024 |
| 89 | 102,46 | 89 | 55,416 | 89 | 33,656 | 89 | 23,628 | 89 | 18,368 |
| 95 | 103,8 | 96 | 58,032 | 96 | 35,488 | 96 | 25,02 | 96 | 19,624 |
| 102 | 105,252 | 103 | 60,316 | 103 | 37,232 | 103 | 26,372 | 103 | 20,764 |
| 109 | 106,636 | 110 | 62,72 | 110 | 38,904 | 110 | 27,908 | 110 | 21,992 |
| 116 | 107,724 | 117 | 65,24 | 117 | 40,712 | 117 | 29,292 | 117 | 23,416 |
| 123 | 109,012 | 123 | 67,476 | 123 | 42,456 | 123 | 30,776 | 123 | 24,636 |
| 129 | 110,108 | 130 | 69,84 | 130 | 44,172 | 130 | 32,236 | 130 | 25,92 |
| 136 | 111,12 | 137 | 72,416 | 137 | 46,076 | 137 | 33,876 | 137 | 27,392 |
| 143 | 112,132 | 144 | 75,2 | 144 | 48,24 | 144 | 35,66 | 144 | 29,136 |
| 150 | 112,916 | 151 | 77,74 | 151 | 50,496 | 151 | 37,536 | 151 | 30,708 |
| 157 | 113,716 | 158 | 80,572 | 158 | 52,76 | 158 | 39,524 | 158 | 32,552 |
| 164 | 114,388 | 166 | 83,588 | 166 | 54,908 | 166 | 41,716 | 166 | 34,392 |
| 172 | 115,144 | 174 | 86,092 | 174 | 57,064 | 174 | 43,652 | 174 | 36,192 |
| 179 | 115,836 | 182 | 88,844 | 182 | 59,344 | 182 | 45,652 | 182 | 38,284 |
| 186 | 116,424 | 190 | 91,396 | 190 | 61,548 | 190 | 47,844 | 190 | 40,276 |
| 193 | 116,996 | 198 | 93,296 | 198 | 63,556 | 198 | 49,748 | 198 | 41,972 |
| 200 | 117,58 | 206 | 95,316 | 206 | 65,888 | 206 | 51,756 | 206 | 43,804 |
| 206 | 118,076 | 214 | 97,332 | 214 | 68,016 | 214 | 53,556 | 214 | 45,716 |
| 213 | 118,544 | 222 | 99,208 | 222 | 70,008 | 222 | 55,508 | 222 | 47,508 |
| 220 | 119,084 | 230 | 100,72 | 230 | 71,656 | 230 | 56,964 | 230 | 49,032 |
| 227 | 119,608 | 238 | 102,11 2 | 238 | 73,456 | 238 | 58,608 | 238 | 50,604 |
| 234 | 120,112 | 246 | 103,34 8 | 246 | 75,056 | 246 | 60,064 | 246 | 51,94 |
| 241 | 120,616 | 254 | 104,76 8 | 254 | 77,064 | 254 | 61,8 | 254 | 53,472 |
| 249 | 121,18 | 262 | 106,27 6 | 262 | 78,3 | 262 | 63,384 | 262 | 54,844 |
| 257 | 121,608 | 270 | 107,92 | 270 | 79,504 | 270 | 65,028 | 270 | 56,256 |
| 265 | 122,044 | 278 | 109,51 6 | 278 | 80,676 | 278 | 66,764 | 278 | 57,84 |
| 273 | 122,384 | | | | | | | | |
| 281 | 122,676 | | | | | | | | |
| 289 | 122,988 | | | | | | | | |
| 297 | 123,316 | | | | | | | | |
| 305 | 123,536 | | | | | | | | |
| 313 | 123,764 | | | | | | | | |
| 321 | 123,92 | | | | | | | | |
| 329 | 124,12 | | | | | | | | |
| 337 | 124,24 8 | | | | | | | | |

| Zeit (h) | Stent 1 Anteil (Gew%) | Zeit (h) | Stent 2 Anteil (Gew%) | Zeit (h) | Stent 3 Anteil (Gew%) | Zeit (h) | Stent 4 Anteil (Gew%) | Zeit (h) | Stent 5 Anteil (Gew% |
|---|---|---|---|---|---|---|---|---|---|
| | | | | | | | | | |
| 0 | 0,00 | 0 | 0 | 0 | 0 | 0 | 0 | 0 | 0 |
| 0,33 | 6,20 | 0,33 | 1,13 | 0,33 | 0,44 | 0,33 | 0,12 | 0,33 | 0,10 |
| 0,67 | 9,03 | 0,67 | 1,55 | 0,67 | 0,64 | 0,67 | 0,19 | 0,67 | 0,17 |
| 1 | 11,29 | 1 | 1,87 | 1 | 0,76 | 1 | 0,29 | 1 | 0,24 |
| 1,5 | 14,27 | 1,5 | 2,31 | 1,5 | 0,99 | 1,5 | 0,39 | 1,5 | 0,33 |
| 2 | 16,39 | 2 | 2,86 | 2 | 1,25 | 2 | 0,52 | 2 | 0,42 |
| 3 | 20,25 | 3 | 3,67 | 3 | 1,65 | 3 | 0,71 | 3 | 0,56 |
| 4 | 23,40 | 4 | 4,39 | 4 | 2,04 | 4 | 0,91 | 4 | 0,73 |
| 5 | 25,94 | 5 | 5,09 | 5 | 2,40 | 5 | 1,10 | 5 | 0,88 |
| 6 | 28,14 | 6 | 5,74 | 6 | 2,76 | 6 | 1,32 | 6 | 1,02 |
| 7 | 30,16 | 7 | 6,37 | 7 | 3,12 | 7 | 1,50 | 7 | 1,18 |
| 9 | 33,51 | 9 | 7,51 | 9 | 3,80 | 9 | 1,87 | 9 | 1,48 |
| 11 | 36,47 | 11 | 8,65 | 11 | 4,48 | 11 | 2,25 | 11 | 1,79 |
| 13 | 39,05 | 13 | 9,67 | 13 | 5,08 | 13 | 2,63 | 13 | 2,07 |
| 16 | 41,92 | 16 | 11,23 | 16 | 5,97 | 16 | 3,22 | 16 | 2,48 |
| 19 | 44,58 | 19 | 12,64 | 19 | 6,81 | 19 | 3,79 | 19 | 2,93 |
| 22 | 46,76 | 22 | 14,07 | 22 | 7,72 | 22 | 4,36 | 22 | 3,41 |
| 26 | 49,31 | 27 | 16,21 | 27 | 8,90 | 27 | 5,17 | 27 | 4,06 |
| 32 | 51,76 | 33 | 18,28 | 33 | 10,14 | 33 | 6,06 | 33 | 4,82 |
| 39 | 54,01 | 40 | 20,78 | 40 | 11,68 | 40 | 7,21 | 40 | 5,71 |
| 47 | 56,38 | 46 | 22,89 | 46 | 13,09 | 46 | 8,26 | 46 | 6,58 |
| 54 | 58,13 | 53 | 24,86 | 53 | 14,55 | 53 | 9,28 | 53 | 7,49 |
| 61 | 59,69 | 60 | 27,00 | 60 | 16,00 | 60 | 10,42 | 60 | 8,37 |
| 68 | 61,12 | 67 | 28,75 | 67 | 17,20 | 67 | 11,37 | 67 | 9,13 |
| 75 | 62,36 | 74 | 30,54 | 74 | 18,49 | 74 | 12,20 | 74 | 10,03 |
| 82 | 63,57 | 81 | 32,38 | 81 | 19,79 | 81 | 13,11 | 81 | 10,83 |
| 89 | 64,56 | 89 | 34,18 | 89 | 21,11 | 89 | 14,15 | 89 | 11,68 |
| 95 | 65,41 | 96 | 35,79 | 96 | 22,26 | 96 | 14,99 | 96 | 12,48 |
| 102 | 66,32 | 103 | 37,20 | 103 | 23,35 | 103 | 15,80 | 103 | 13,21 |
| 109 | 67,19 | 110 | 38,68 | 110 | 24,40 | 110 | 16,72 | 110 | 13,99 |
| 116 | 67,88 | 117 | 40,23 | 117 | 25,53 | 117 | 17,55 | 117 | 14,90 |
| 123 | 68,69 | 123 | 41,61 | 123 | 26,63 | 123 | 18,43 | 123 | 15,67 |
| 129 | 69,38 | 130 | 43,07 | 130 | 27,70 | 130 | 19,31 | 130 | 16,49 |
| 136 | 70,02 | 137 | 44,66 | 137 | 28,90 | 137 | 20,29 | 137 | 17,42 |
| 143 | 70,66 | 144 | 46,38 | 144 | 30,25 | 144 | 21,36 | 144 | 18,53 |
| 150 | 71,15 | 151 | 47,94 | 151 | 31,67 | 151 | 22,48 | 151 | 19,53 |
| 157 | 71,65 | 158 | 49,69 | 158 | 33,09 | 158 | 23,67 | 158 | 20,71 |
| 164 | 72,08 | 166 | 51,55 | 166 | 34,44 | 166 | 24,99 | 166 | 21,88 |
| 172 | 72,55 | 174 | 53,09 | 174 | 35,79 | 174 | 26,15 | 174 | 23,02 |
| 179 | 72,99 | 182 | 54,79 | 182 | 37,22 | 182 | 27,34 | 182 | 24,35 |
| 186 | 73,36 | 190 | 56,37 | 190 | 38,60 | 190 | 28,66 | 190 | 25,62 |
| 193 | 73,72 | 198 | 57,54 | 198 | 39,86 | 198 | 29,80 | 198 | 26,70 |
| 200 | 74,09 | 206 | 58,78 | 206 | 41,32 | 206 | 31,00 | 206 | 27,87 |
| 206 | 74,40 | 214 | 60,03 | 214 | 42,66 | 214 | 32,08 | 214 | 29,08 |
| 213 | 74,70 | 222 | 61,18 | 222 | 43,91 | 222 | 33,25 | 222 | 30,22 |
| 220 | 75,04 | 230 | 62,12 | 230 | 44,94 | 230 | 34,12 | 230 | 31,19 |
| 227 | 75,37 | 238 | 62,97 | 238 | 46,07 | 238 | 35,11 | 238 | 32,19 |
| 234 | 75,68 | 246 | 63,74 | 246 | 47,07 | 246 | 35,98 | 246 | 33,04 |
| 241 | 76,00 | 254 | 64,61 | 254 | 48,33 | 254 | 37,02 | 254 | 34,02 |
| 249 | 76,36 | 262 | 65,54 | 262 | 49,11 | 262 | 37,97 | 262 | 34,89 |
| 257 | 76,63 | 270 | 66,56 | 270 | 49,86 | 270 | 38,95 | 270 | 35,79 |
| 265 | 76,90 | 278 | 67,54 | 278 | 50,60 | 278 | 39,99 | 278 | 36,79 |
| 273 | 77,12 | | | | | | | | |
| 281 | 77,30 | | | | | | | | |
| 289 | 77,50 | | | | | | | | |
| 297 | 77,70 | | | | | | | | |
| 305 | 77,84 | | | | | | | | |
| 313 | 77,99 | | | | | | | | |
| 321 | 78,08 | | | | | | | | |
| 329 | 78,21 | | | | | | | | |
| 337 | 78,29 | | | | | | | | |

### 2. Stent mit Beschichtung aus Polyurethan des Beispiels 3 (Vergleich)

| | Grundbeschichtung (µg) | Sirolimus (µg) | Deckbeschichtung (µg) |
|---|---|---|---|
| Stent 1 | 1141 | 171,15 | 0 |
| Stent 2 | 1096 | 164,4 | 127 |
| Stent 3 | 1091 | 163,65 | 329 |
| Stent 4 | 1113 | 166,95 | 504 |
| Stent 5 | 1103 | 165,45 | 704 |

| Zeit (h) | Stent 1 Summe abs. (µg) | Zeit (h) | Stent 2 Summe abs. (µg) | Zeit (h) | Stent 3 Summe abs. (µg) | Zeit (h) | Stent 4 Summe abs. (µg) | Zeit (h) | Stent 5 Summe abs. (µg) |
|---|---|---|---|---|---|---|---|---|---|
| | | | | | | | | | |
| 0,0 | 0 | 0 | 0 | 0 | 0 | 0 | 0 | 0 | 0 |
| 0,33 | 15,876 | 0,33 | 13,388 | 0,33 | 11,548 | 0,33 | 10,82 | 0,33 | 10,432 |
| 0,67 | 26,62 | 0,67 | 22,248 | 0,67 | 19,344 | 0,67 | 18,868 | 0,67 | 17,872 |
| 1 | 34,624 | 1 | 29,824 | 1 | 26,856 | 1 | 26,084 | 1 | 24,796 |
| 2 | 44,412 | 1,5 | 39,252 | 1,5 | 35,612 | 1,5 | 34,556 | 1,5 | 33,196 |
| 2 | 52,928 | 2 | 47,984 | 2 | 43,228 | 2 | 42,156 | 2 | 41,112 |
| 3 | 66,548 | 3 | 60,228 | 3 | 54,372 | 3 | 53,228 | 3 | 52,032 |
| 4 | 78,356 | 4 | 71,988 | 4 | 64,796 | 4 | 63,868 | 4 | 61,964 |
| 5 | 88,336 | 5 | 81,936 | 5 | 74,188 | 5 | 73,328 | 5 | 71,1 |
| 6 | 96,336 | 6 | 90,844 | 6 | 82,5 | 6 | 81,916 | 6 | 79,116 |
| 7 | 103,124 | 7 | 98,348 | 7 | 90,348 | 7 | 89,748 | 7 | 86,616 |
| 9 | 111,092 | 9 | 106,58 8 | 9 | 99,056 | 9 | 98,784 | 9 | 95,28 |
| 11 | 116,756 | 11 | 112,28 8 | 11 | 105,356 | 11 | 105,848 | 11 | 102,52 |
| 13 | 120,684 | 13 | 116,13 6 | 13 | 109,812 | 13 | 111,044 | 13 | 107,992 |
| 16 | 124,204 | 16 | 118,8 | 16 | 115,004 | 16 | 114,948 | 16 | 112,004 |
| 19 | 126,816 | 19 | 121,39 6 | 19 | 117,224 | 19 | 116,668 | 19 | 116,068 |
| 22 | 128,784 | 22 | 122,53 6 | 22 | 120,196 | 22 | 118,208 | 22 | 118,096 |
| 26 | 130,452 | 26 | 123,38 8 | 26 | 122,82 | 26 | 119,492 | 26 | 119,524 |
| 32 | 131,876 | 32 | 123,88 4 | 32 | 124,696 | 32 | 120,312 | 32 | 120,52 |
| 39 | 132,96 | 39 | 124,26 8 | 39 | 126,136 | 39 | 120,872 | 39 | 121,176 |
| 46 | 133,58 | 46 | 124,46 8 | 46 | 127,136 | 46 | 121,212 | 46 | 121,608 |
| 53 | 133,924 | 52 | 124,62 4 | 52 | 127,8 | 52 | 121,432 | 52 | 121,892 |
| 60 | 134,088 | 59 | 124,69 6 | 59 | 128,352 | 59 | 121,6 | 59 | 122,088 |
| 67 | 134,204 | 66 | 124,74 4 | 66 | 128,72 | 66 | 121,736 | 66 | 122,24 |
| 74 | 134,284 | 73 | 124,77 2 | 73 | 128,984 | 73 | 121,804 | 73 | 122,316 |
| 81 | 134,34 | 80 | 124,77 2 | 80 | 129,176 | 80 | 121,892 | 80 | 122,384 |

| Zeit (h) | Stent 1 Anteil (Gew%) | Zeit (h) | Stent 2 Anteil (Gew%) | Zeit (h) | Stent 3 Anteil (Gew%) | Zeit (h) | Stent 4 Anteil (Gew%) | Zeit (h) | Stent 5 Anteil (Gew%) |
|---|---|---|---|---|---|---|---|---|---|
| | | | | | | | | | |
| 0 | 0 | 0 | 0 | 0 | 0 | 0 | 0 | 0 | 0 |
| 0,33 | 9,28 | 0,33 | 8,14 | 0,33 | 7,06 | 0,33 | 6,48 | 0,33 | 6,31 |
| 0,67 | 15,55 | 0,67 | 13,53 | 0,67 | 11,82 | 0,67 | 11,30 | 0,67 | 10,80 |
| 1 | 20,23 | 1 | 18,14 | 1 | 16,41 | 1 | 15,62 | 1 | 14,99 |
| 2 | 25,95 | 2 | 23,88 | 2 | 21,76 | 2 | 20,70 | 2 | 20,06 |
| 2 | 30,92 | 2 | 29,19 | 2 | 26,41 | 2 | 25,25 | 2 | 24,85 |
| 3 | 38,88 | 3 | 36,64 | 3 | 33,22 | 3 | 31,88 | 3 | 31,45 |
| 4 | 45,78 | 4 | 43,79 | 4 | 39,59 | 4 | 38,26 | 4 | 37,45 |
| 5 | 51,61 | 5 | 49,84 | 5 | 45,33 | 5 | 43,92 | 5 | 42,97 |
| 6 | 56,29 | 6 | 55,26 | 6 | 50,41 | 6 | 49,07 | 6 | 47,82 |
| 7 | 60,25 | 7 | 59,82 | 7 | 55,21 | 7 | 53,76 | 7 | 52,35 |
| 9 | 64,91 | 9 | 64,83 | 9 | 60,53 | 9 | 59,17 | 9 | 57,59 |
| 11 | 68,22 | 11 | 68,30 | 11 | 64,38 | 11 | 63,40 | 11 | 61,96 |
| 13 | 70,51 | 13 | 70,64 | 13 | 67,10 | 13 | 66,51 | 13 | 65,27 |
| 16 | 72,57 | 16 | 72,26 | 16 | 70,27 | 16 | 68,85 | 16 | 67,70 |
| 19 | 74,10 | 19 | 73,84 | 19 | 71,63 | 19 | 69,88 | 19 | 70,15 |
| 22 | 75,25 | 22 | 74,54 | 22 | 73,45 | 22 | 70,80 | 22 | 71,38 |
| 26 | 76,22 | 26 | 75,05 | 26 | 75,05 | 26 | 71,57 | 26 | 72,24 |
| 32 | 77,05 | 32 | 75,36 | 32 | 76,20 | 32 | 72,06 | 32 | 72,84 |
| 39 | 77,69 | 39 | 75,59 | 39 | 77,08 | 39 | 72,40 | 39 | 73,24 |
| 46 | 78,05 | 46 | 75,71 | 46 | 77,69 | 46 | 72,60 | 46 | 73,50 |
| 53 | 78,25 | 52 | 75,81 | 52 | 78,09 | 52 | 72,74 | 52 | 73,67 |
| 60 | 78,35 | 59 | 75,85 | 59 | 78,43 | 59 | 72,84 | 59 | 73,79 |
| 67 | 78,41 | 66 | 75,88 | 66 | 78,66 | 66 | 72,92 | 66 | 73,88 |
| 74 | 78,46 | 73 | 75,90 | 73 | 78,82 | 73 | 72,96 | 73 | 73,93 |
| 81 | 78,49 | 80 | 75,90 | 80 | 78,93 | 80 | 73,01 | 80 | 73,97 |

### 3. Stent mit Beschichtung aus Polyurethan des Beispiels 4 (Vergleich)

| | Grundbeschichtung (µg) | Sirolimus (µg) | Deckbeschichtung (µg) |
|---|---|---|---|
| Stent 1 | 1162 | 174,3 | 0 |
| Stent 2 | 1114 | 167,1 | 108 |
| Stent 3 | 1139 | 170,85 | 322 |
| Stent 4 | 1112 | 166,8 | 520 |
| Stent 5 | 1109 | 166,35 | 723 |

| Zeit (h) | Stent 1 Summe abs. (µg) | Zeit (h) | Stent 2 Summe abs. (µg) | Zeit (h) | Stent 3 Summe abs. (µg) | Zeit (h) | Stent 4 Summe abs. (µg) | Zeit (h) | Stent 5 Summe abs. (µg) |
|---|---|---|---|---|---|---|---|---|---|
| | | | | | | | | | |
| 0 | 0 | 0 | 0 | 0 | 0 | 0 | 0 | 0 | 0 |
| 0,33 | 22,156 | 0,33 | 14,376 | 0,33 | 12,476 | 0,33 | 10,74 | 0,33 | 9,352 |
| 0,67 | 32,9 | 0,67 | 24,152 | 0,67 | 21,212 | 0,67 | 17,612 | 0,67 | 15,956 |
| 1 | 44,528 | 1 | 32,756 | 1 | 29,192 | 1 | 24,176 | 1 | 21,54 |
| 1,5 | 59,508 | 1,5 | 43,06 | 1,5 | 38,288 | 1,5 | 32,004 | 1,5 | 28,3 |
| 2 | 71,688 | 2 | 52,88 | 2 | 47,228 | 2 | 39,756 | 2 | 34,968 |
| 3 | 87,868 | 3 | 65,664 | 3 | 59,028 | 3 | 50,448 | 3 | 44,032 |
| 4 | 100,376 | 4 | 75,876 | 4 | 69,124 | 4 | 59,776 | 4 | 52,188 |
| 5 | 109,432 | 5 | 84,2 | 5 | 77,776 | 5 | 68,244 | 5 | 60,076 |
| 6 | 116,072 | 6 | 90,14 | 6 | 84,74 | 6 | 75,12 | 6 | 66,388 |
| 7 | 121,048 | 7 | 94,872 | 7 | 90,616 | 7 | 81,064 | 7 | 71,98 |
| 9 | 125,836 | 9 | 99,176 | 9 | 96,28 | 9 | 87,412 | 9 | 78,356 |
| 11 | 129,5 | 11 | 102,072 | 11 | 100,608 | 11 | 92,076 | 11 | 83,912 |
| 13 | 132,48 | 13 | 105,092 | 13 | 102,632 | 13 | 95,848 | 13 | 88,084 |
| 16 | 135,4 | 15 | 107,336 | 15 | 104,008 | 15 | 98,66 | 15 | 91,48 |
| 19 | 137,808 | 17 | 108,948 | 17 | 104,944 | 17 | 100,876 | 17 | 94,116 |
| 22 | 139,712 | 20 | 110,316 | 20 | 105,608 | 20 | 105,548 | 20 | 99,024 |
| 26 | 141,336 | 24 | 111,196 | 24 | 106,048 | 24 | 106,992 | 24 | 101,064 |
| 32 | 142,544 | 30 | 111,82 | 30 | 106,336 | 30 | 107,96 | 30 | 102,612 |
| 39 | 143,332 | 37 | 112,228 | 37 | 106,496 | 37 | 108,64 | 37 | 103,768 |
| 46 | 143,856 | 44 | 112,488 | 44 | 106,644 | 44 | 109,1 | 44 | 104,556 |
| 53 | 144,092 | 50 | 112,652 | 50 | 106,728 | 50 | 109,404 | 50 | 105,048 |
| 60 | 144,252 | 57 | 112,796 | 57 | 106,772 | 57 | 109,596 | 57 | 105,396 |
| 67 | 144,372 | 64 | 112,872 | 64 | 106,808 | 64 | 109,716 | 64 | 105,636 |
| 74 | 144,44 | 71 | 112,944 | 71 | 106,852 | 71 | 109,828 | 71 | 105,832 |
| 81 | 144,46 | 78 | 112,964 | 78 | 106,876 | 78 | 109,868 | 78 | 105,972 |

| Zeit (h) | Stent 1 Anteil (Gew%) | Zeit (h) | Stent 2 Anteil (Gew%) | Zeit (h) | Stent 3 Anteil (Gew%) | Zeit (h) | Stent 4 Anteil (Gew%) | Zeit (h) | Stent 5 Anteil (Gew%) |
|---|---|---|---|---|---|---|---|---|---|
| | | | | | | | | | |
| 0 | 0 | 0 | 0 | 0 | 0 | 0 | 0 | 0 | 0 |
| 0,33 | 12,71 | 0,33 | 8,60 | 0,33 | 7,30 | 0,33 | 6,44 | 0,33 | 5,62 |
| 0,67 | 18,88 | 0.67 | 14,45 | 0,67 | 12,42 | 0,67 | 10,56 | 0,67 | 9,59 |
| 1 | 25,55 | 1 | 19,60 | 1 | 17,09 | 1 | 14,49 | 1 | 12,95 |
| 1,5 | 34,14 | 2 | 25,77 | 2 | 22,41 | 2 | 19,19 | 2 | 17,01 |
| 2 | 41,13 | 2 | 31,65 | 2 | 27,64 | 2 | 23,83 | 2 | 21,02 |
| 3 | 50,41 | 3 | 39,30 | 3 | 34,55 | 3 | 30,24 | 3 | 26,47 |
| 4 | 57,59 | 4 | 45,41 | 4 | 40,46 | 4 | 35,84 | 4 | 31,37 |
| 5 | 62,78 | 5 | 50,39 | 5 | 45,52 | 5 | 40,91 | 5 | 36,11 |
| 6 | 66,59 | 6 | 53,94 | 6 | 49,60 | 6 | 45,04 | 6 | 39,91 |
| 7 | 69,45 | 7 | 56,78 | 7 | 53,04 | 7 | 48,60 | 7 | 43,27 |
| 9 | 72,20 | 9 | 59,35 | 9 | 56,35 | 9 | 52,41 | 9 | 47,10 |
| 11 | 74,30 | 11 | 61,08 | 11 | 58,89 | 11 | 55,20 | 11 | 50,44 |
| 13 | 76,01 | 13 | 62,89 | 13 | 60,07 | 13 | 57,46 | 13 | 52,95 |
| 16 | 77,68 | 15 | 64,23 | 15 | 60,88 | 15 | 59,15 | 15 | 54,99 |
| 19 | 79,06 | 17 | 65,20 | 17 | 61,42 | 17 | 60,48 | 17 | 56,58 |
| 22 | 80,16 | 20 | 66,02 | 20 | 61,81 | 20 | 63,28 | 20 | 59,53 |
| 26 | 81,09 | 24 | 66,54 | 24 | 62,07 | 24 | 64,14 | 24 | 60,75 |
| 32 | 81,78 | 30 | 66,92 | 30 | 62,24 | 30 | 64,72 | 30 | 61,68 |
| 39 | 82,23 | 37 | 67,16 | 37 | 62,33 | 37 | 65,13 | 37 | 62,38 |
| 46 | 82,53 | 44 | 67,32 | 44 | 62,42 | 44 | 65,41 | 44 | 62,85 |
| 53 | 82,67 | 50 | 67,42 | 50 | 62,47 | 50 | 65,59 | 50 | 63,15 |
| 60 | 82,76 | 57 | 67,50 | 57 | 62,49 | 57 | 65,71 | 57 | 63,36 |
| 67 | 82,83 | 64 | 67,55 | 64 | 62,52 | 64 | 65,78 | 64 | 63,50 |
| 74 | 82,87 | 71 | 67,59 | 71 | 62,54 | 71 | 65,84 | 71 | 63,62 |
| 81 | 82,88 | 78 | 67,60 | 78 | 62,56 | 78 | 65,87 | 78 | 63,70 |

### 4. Stent mit Beschichtung aus Polyurethan des Beispiels 5 (Vergleich)

| | Grundbeschichtung (µg) | Sirolimus (µg) | Deckbeschichtung (µg) |
|---|---|---|---|
| Stent 1 | 1100 | 165 | 0 |
| Stent 2 | 1076 | 161,4 | 105 |
| Stent 3 | 1107 | 166,05 | 317 |
| Stent 4 | 1125 | 168,75 | 528 |
| Stent 5 | 1092 | 163,8 | 731 |

| Zeit (h) | Stent 1 Summe abs. (µg) | Zeit (h) | Stent 2 Summe abs. (µg) | Zeit (h) | Stent 3 Summe abs. (µg) | Zeit (h) | Stent 4 Summe abs. (µg) | Zeit (h) | Stent 5 Summe abs. (µg) |
|---|---|---|---|---|---|---|---|---|---|
| | | | | | | | | | |
| 0 | 0 | 0 | 0 | 0 | 0 | 0 | 0 | 0 | 0 |
| 0,33 | 12,06 | 0,33 | 12,508 | 0,33 | 10,452 | 0,33 | 11,168 | 0,33 | 9,544 |
| 0,67 | 17,456 | 0,67 | 21,228 | 0,67 | 13,528 | 0,67 | 17,648 | 0,67 | 15,964 |
| 1 | 21,448 | 1 | 29,432 | 1 | 20,116 | 1 | 23,676 | 1 | 21,84 |
| 1,5 | 26,98 | 1,5 | 39,232 | 1,5 | 27,664 | 1,5 | 30,64 | 1,5 | 27,992 |
| 2 | 31,616 | 2 | 47,26 | 2 | 34,688 | 2 | 37,316 | 2 | 34,032 |
| 3 | 39,736 | 3 | 58,892 | 3 | 44,792 | 3 | 47,708 | 3 | 42,816 |
| 4 | 46,876 | 4 | 69,048 | 4 | 53,908 | 4 | 56,572 | 4 | 51,084 |
| 5 | 52,672 | 5 | 77,824 | 5 | 62,288 | 5 | 64,796 | 5 | 58,692 |
| 6 | 58,244 | 6 | 85,344 | 6 | 69,76 | 6 | 72,06 | 6 | 65,316 |
| 7 | 63,268 | 7 | 91,856 | 7 | 76,5 | 7 | 78,912 | 7 | 71,288 |
| 9 | 71,652 | 9 | 99,976 | 9 | 85,712 | 9 | 87,468 | 9 | 78,92 |
| 11 | 78,852 | 11 | 106,62 | 11 | 93,788 | 11 | 94,804 | 11 | 86,088 |
| 13 | 84,968 | 13 | 111,544 | 13 | 99,752 | 13 | 100,548 | 13 | 93,152 |
| 16 | 92,912 | 16 | 115,092 | 16 | 102,992 | 15 | 105,304 | 15 | 97,936 |
| 19 | 100,072 | 19 | 117,628 | 19 | 105,904 | 17 | 109,708 | 17 | 101,964 |
| 22 | 105,332 | 22 | 119,46 | 22 | 107,368 | 20 | 111,716 | 20 | 104,704 |
| 26 | 110,788 | 26 | 121,06 | 26 | 108,404 | 24 | 115,696 | 24 | 108,412 |
| 32 | 115,772 | 32 | 122,24 | 32 | 109,052 | 30 | 119,196 | 30 | 111,408 |
| 39 | 120,584 | 39 | 122,692 | 39 | 109,916 | 37 | 122,14 | 37 | 113,74 |
| 46 | 123,676 | 46 | 123,32 | 46 | 110,172 | 44 | 124,128 | 44 | 115,28 |
| 53 | 126,168 | 52 | 123,712 | 52 | 110,344 | 50 | 125,564 | 50 | 116,284 |
| 60 | 127,828 | 59 | 124,072 | 59 | 110,424 | 57 | 126,728 | 57 | 117,112 |
| 67 | 129,076 | 66 | 124,352 | 66 | 110,54 | 64 | 127,68 | 64 | 117,804 |
| 74 | 129,884 | 73 | 124,544 | 73 | 110,58 | 71 | 128,424 | 71 | 118,24 |
| 81 | 130,556 | 80 | 124,732 | 80 | 110,624 | 78 | 128,96 | 78 | 118,596 |
| 88 | 130,988 | | | | | 84 | 129,392 | 84 | 118,88 |
| 95 | 131,368 | | | | | 91 | 129,648 | 91 | 119,064 |
| 102 | 131,692 | | | | | 98 | 129,916 | 98 | 119,256 |
| 109 | 131,932 | | | | | 105 | 130,036 | 105 | 119,396 |
| 116 | 132,12 | | | | | 112 | 130,2 | 112 | 119,492 |
| 123 | 132,264 | | | | | | | | |
| 130 | 132,348 | | | | | | | | |
| 137 | 132,46 | | | | | | | | |
| 144 | 132,54 | | | | | | | | |

| Zeit (h) | Stent 1 Anteil (Gew%) | Zeit (h) | Stent 2 Anteil (Gew%) | Zeit (h) | Stent 3 Anteil (Gew%) | Zeit (h) | Stent 4 Anteil (Gew%) | Zeit (h) | Stent 5 Anteil (Gew %) |
|---|---|---|---|---|---|---|---|---|---|
| | | | | | | | | | |
| 0 | 0 | 0 | 0 | 0 | 0 | 0 | 0 | 0 | 0 |
| 0,33 | 7,31 | 0,33 | 7,75 | 0,33 | 6,29 | 0,33 | 6,62 | 0,33 | 5,83 |
| 0,67 | 10,58 | 0,67 | 13,15 | 0,67 | 8,15 | 0,67 | 10,46 | 0,67 | 9,75 |
| 1 | 13,00 | 1 | 18,24 | 1 | 12,11 | 1 | 14,03 | 1 | 13,33 |
| 1,5 | 16,35 | 1,5 | 24,31 | 1,5 | 16,66 | 1,5 | 18,16 | 1,5 | 17,09 |
| 2 | 19,16 | 2 | 29,28 | 2 | 20,89 | 2 | 22,11 | 2 | 20,78 |
| 3 | 24,08 | 3 | 36,49 | 3 | 26,98 | 3 | 28,27 | 3 | 26,14 |
| 4 | 28,41 | 4 | 42,78 | 4 | 32,46 | 4 | 33,52 | 4 | 31,19 |
| 5 | 31,92 | 5 | 48,22 | 5 | 37,51 | 5 | 38,40 | 5 | 35,83 |
| 6 | 35,30 | 6 | 52,88 | 6 | 42,01 | 6 | 42,70 | 6 | 39,88 |
| 7 | 38,34 | 7 | 56,91 | 7 | 46,07 | 7 | 46,76 | 7 | 43,52 |
| 9 | 43,43 | 9 | 61,94 | 9 | 51,62 | 9 | 51,83 | 9 | 48,18 |
| 11 | 47,79 | 11 | 66,06 | 11 | 56,48 | 11 | 56,18 | 11 | 52,56 |
| 13 | 51,50 | 13 | 69,11 | 13 | 60,07 | 13 | 59,58 | 13 | 56,87 |
| 16 | 56,31 | 16 | 71,31 | 16 | 62,02 | 15 | 62,40 | 15 | 59,79 |
| 19 | 60,65 | 19 | 72,88 | 19 | 63,78 | 17 | 65,01 | 17 | 62,25 |
| 22 | 63,84 | 22 | 74,01 | 22 | 64,66 | 20 | 66,20 | 20 | 63,92 |
| 26 | 67,14 | 26 | 75,01 | 26 | 65,28 | 24 | 68,56 | 24 | 66,19 |
| 32 | 70,16 | 32 | 75,74 | 32 | 65,67 | 30 | 70,63 | 30 | 68,01 |
| 39 | 73,08 | 39 | 76,02 | 39 | 66,19 | 37 | 72,38 | 37 | 69,44 |
| 46 | 74,96 | 46 | 76,41 | 46 | 66,35 | 44 | 73,56 | 44 | 70,38 |
| 53 | 76,47 | 52 | 76,65 | 52 | 66,45 | 50 | 74,41 | 50 | 70,99 |
| 60 | 77,47 | 59 | 76,87 | 59 | 66,50 | 57 | 75,10 | 57 | 71,50 |
| 67 | 78,23 | 66 | 77,05 | 66 | 66,57 | 64 | 75,66 | 64 | 71,92 |
| 74 | 78,72 | 73 | 77,16 | 73 | 66,59 | 71 | 76,10 | 71 | 72,19 |
| 81 | 79,12 | 80 | 77,28 | 80 | 66,62 | 78 | 76,42 | 78 | 72,40 |
| 88 | 79,39 | | | | | 84 | 76,68 | 84 | 72,58 |
| 95 | 79,62 | | | | | 91 | 76,83 | 91 | 72,69 |
| 102 | 79,81 | | | | | 98 | 76,99 | 98 | 72,81 |
| 109 | 79,96 | | | | | 105 | 77,06 | 105 | 72,89 |
| 116 | 80,07 | | | | | 112 | 77,16 | 112 | 72,95 |
| 123 | 80,16 | | | | | | | | |
| 130 | 80,21 | | | | | | | | |
| 137 | 80,28 | | | | | | | | |
| 144 | 80,33 | | | | | | | | |

### 5. Stent mit Beschichtung aus Polyurethan des Beispiels 6 (Vergleich)

| | Grundbeschichtung (µg) | Sirolimus (µg) | | Deckbeschichtung (µg) |
|---|---|---|---|---|
| Stent 1 | 1138 | 170,7 | | 0 |

| Zeit (h) | Stent 1 (ohne Deckschicht) Summe abs. (µg) | | | |
|---|---|---|---|---|
| | | | | |
| 0 | 0 | | | |
| 0,33 | 26,32 | | | |
| 0,67 | 42,396 | | | |
| 1 | 54,628 | | | |
| 1,5 | 69,044 | | | |
| 2 | 80,432 | | | |
| 3 | 95,484 | | | |
| 4 | 106,52 | | | |
| 5 | 114,588 | | | |
| 6 | 120,588 | | | |
| 7 | 125,24 | | | |
| 9 | 133,22 | | | |
| 11 | 137,94 | | | |
| 13 | 141,832 | | | |
| 16 | 145,364 | | | |
| 19 | 148,132 | | | |
| 22 | 150,256 | | | |
| 25 | 151,82 | | | |
| 29 | 153,208 | | | |
| 35 | 154,424 | | | |
| 42 | 155,156 | | | |
| 49 | 155,712 | | | |
| 56 | 156,044 | | | |
| 63 | 156,252 | | | |
| 70 | 156,452 | | | |
| 78 | 156,584 | | | |

| Zeit (h) | Stent 1 (ohne Deckschicht) Anteil (Gew%) | | | |
|---|---|---|---|---|
| | | | | |
| 0 | 0,00 | | | |
| 0,33 | 15,42 | | | |
| 0,67 | 24,84 | | | |
| 1 | 32,00 | | | |
| 1,5 | 40,45 | | | |
| 2 | 47,12 | | | |
| 3 | 55,94 | | | |
| 4 | 62,40 | | | |
| 5 | 67,13 | | | |
| 6 | 70,64 | | | |
| 7 | 73,37 | | | |
| 9 | 78,04 | | | |
| 11 | 80,81 | | | |
| 13 | 83,09 | | | |
| 16 | 85,16 | | | |
| 19 | 86,78 | | | |
| 22 | 88,02 | | | |
| 25 | 88,94 | | | |
| 29 | 89,75 | | | |
| 35 | 90,47 | | | |
| 42 | 90,89 | | | |
| 49 | 91,22 | | | |
| 56 | 91,41 | | | |
| 63 | 91,54 | | | |
| 70 | 91,65 | | | |
| 78 | 91,73 | | | |

### 6. Diskussion der Ergebnisse

Ziel der Entwicklung war die Herstellung einer Stentbeschichtung, welche über mehrere Wochen in kontinuierlichen kleinen Dosen Wirkstoff aus dem in der Beschichtung vorhandenen Depot abgibt.

Die Rohdaten können in folgender Weise interpretiert werden:

Beispiel 2 (erfinderisch): Die Freisetzung erfolgt über einen langen Zeitraum. Nach 200 h erfolgt immer noch eine kontinuierliche Freisetzung von Sirolimus. Die Beschichtung mit einer wirkstofffreien Polymerschicht über die wirkstoffhaltige Schicht hat einen signifikanten Effekt. Hierdurch wird die Freisetzungsgeschwindigkeit weiter reduziert. Es erfolgt nach mehr als 200 h immer noch eine kontinuierliche Abgabe von Wirkstoff, ohne dass das Wirkstoffdepot aufgebraucht worden ist.

Beispiel 3 (Vergleich): Es erfolgt eine schnelle Freisetzung. Das Wirkstoffdepot ist nach etwa 30 h aufgebraucht. Das Aufbringen einer medikamentenfreien Deckschicht bewirkt keine signifikante Verlangsamung der Freisetzung.

Beispiel 4 (Vergleich): Es erfolgt eine schnelle Freisetzung. Das Wirkstoffdepot ist nach etwa 30 h aufgebraucht. Das Aufbringen einer medikamentenfreien Deckschicht verlangsamt die Freisetzung nur unwesentlich. Die Deckschicht verhindert, dass mehr als 70 % des eingesetzten Wirkstoffes freigesetzt werden.

Beispiel 5 (Vergleich): Es erfolgt eine schnelle Freisetzung. Das Wirkstoffdepot ist nach etwa 30 h aufgebraucht. Das Aufbringen einer medikamentenfreien Deckschicht bewirkt keine signifikante Verlangsamung der Freisetzung.

Beispiel 6 (Vergleich): Es erfolgt eine sehr schnelle Freisetzung. Die Menge an freigesetztem Material ist wesentlich höher als bei allen anderen Stents. Das Wirkstoffdepot ist nach 20 h ausgeschöpft.

Das Wirkstoffdepot ist für alle Vergleichsverbindungen nach maximal 30 h ausgeschöpft.

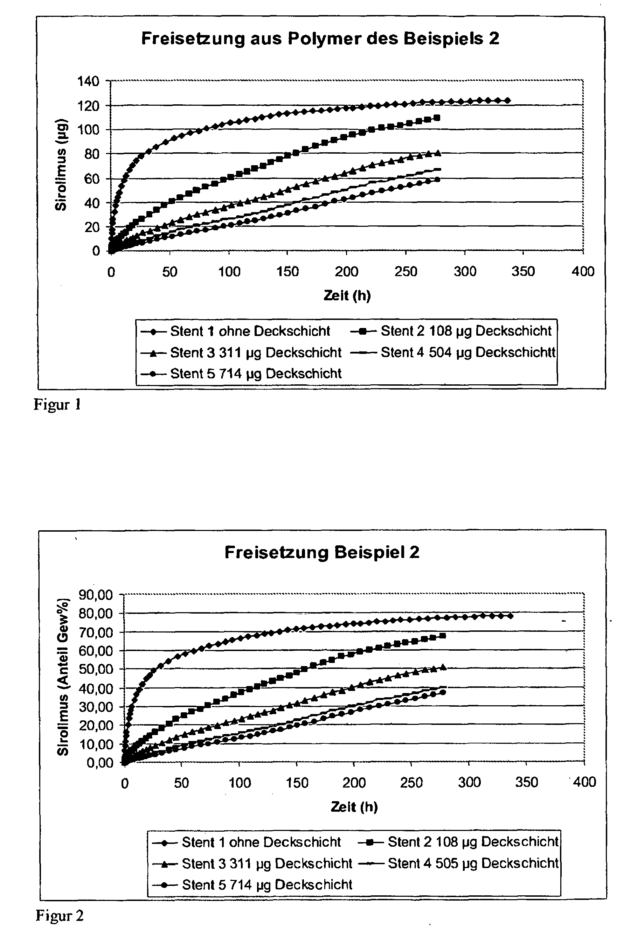

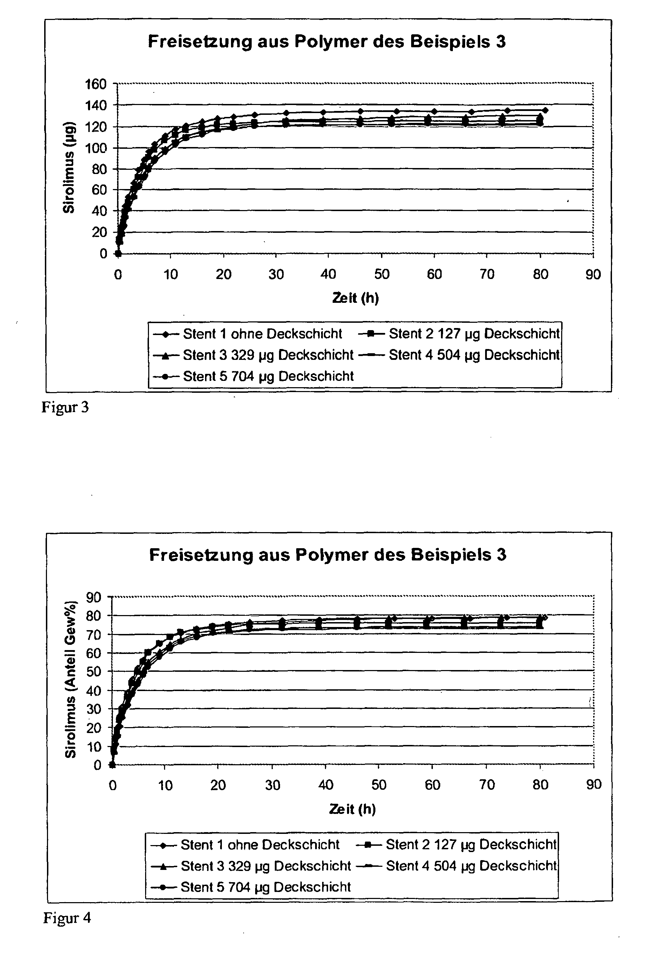

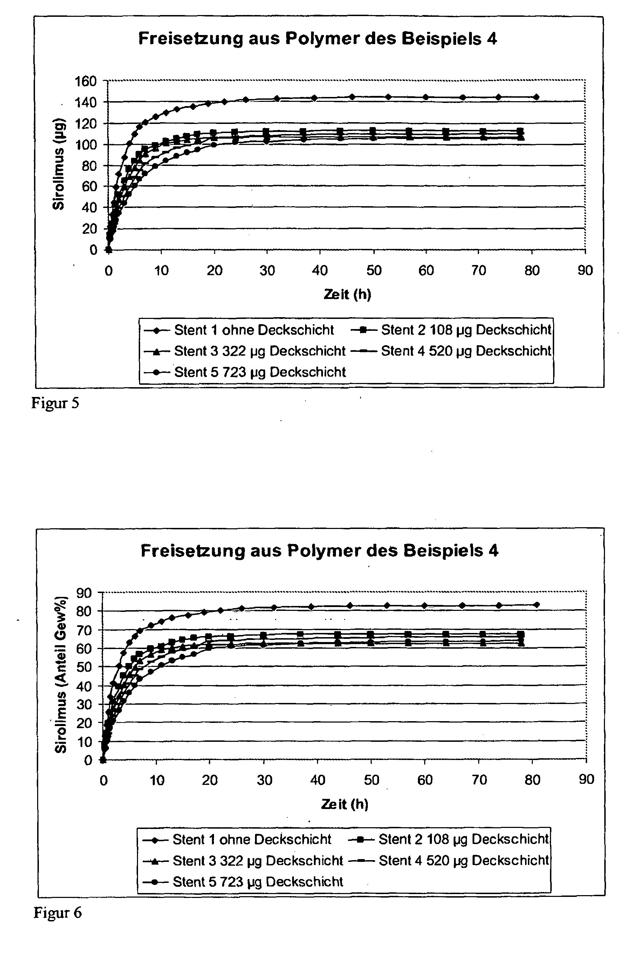

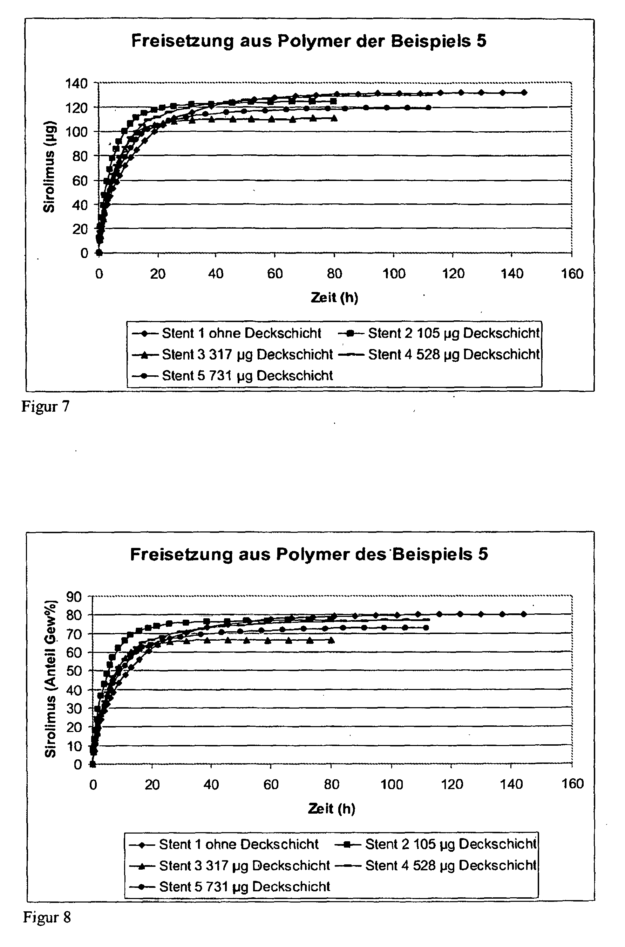

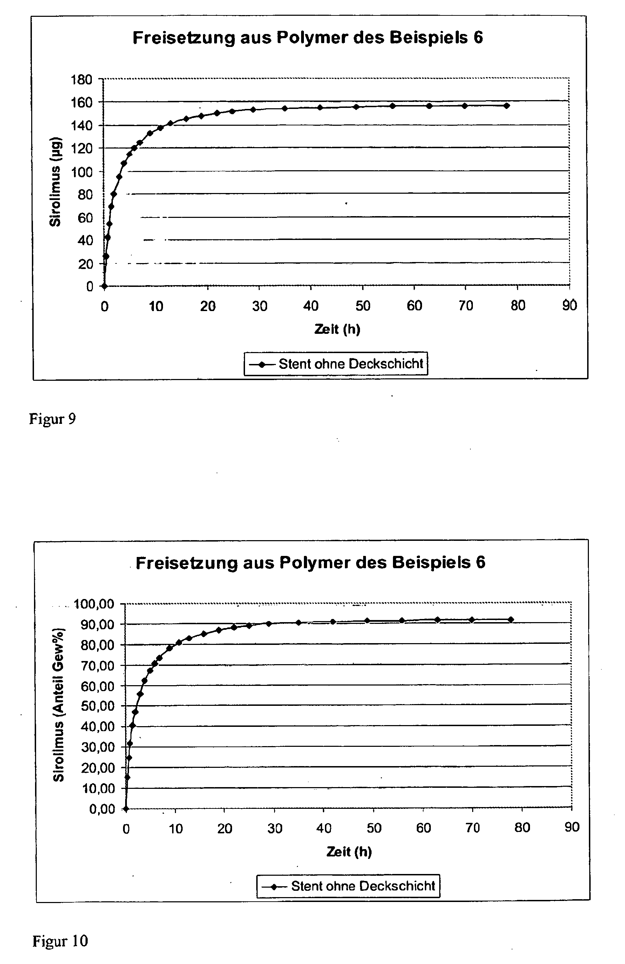

## Patentansprüche

1. Polyurethanharnstoff der Struktureinheiten der Formel (I) 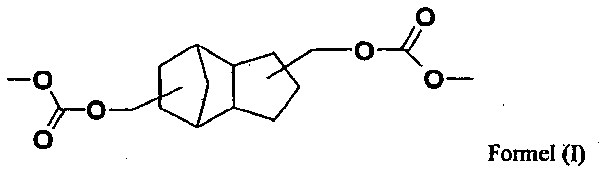 aufweist und nicht mit wenigstens einer Copolymereinheit aus Polyethylenoxid und Polypropylenoxid terminiert ist.

2. Polyurethartharnstoff nach Anspruch 1, **dadurch gekennzeichnet, dass** er auf einer Polycarbonatpolyolkomponente basiert, die bevorzugt eine mittlere Hydroxylfunktionalität von 1,7 bis 2,3 aufweist.

3. Polyurethanharnstoff nach Anspruch 2, **dadurch gekennzeichnet, dass** die Polycarbonatpolyolkomponente Polycarbonatpolyole a1) umfasst, die durch Umsetzung von Kohlensäurederivaten mit difunktionellen Alkoholen der Formel (II) 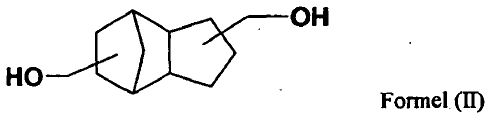 erhältlich sind.

4. Polyurethanharnstoff nach Anspruch 3, **dadurch gekennzeichnet, dass** die Polycarbonatpolyolkomponente neben den Polycarbonatpolyolen a1) weitere Polycarbonatpolyole a2) umfasst.

5. Polyurethanharnstoff nach Anspruch 4, **dadurch gekennzeichnet, dass** es sich bei den Polycarbonatpolyolen a2) um Verbindungen mit einer mittleren Hydroxylfunktionalität von 1,7 bis 2,3 und einem durch die OH-Zahl bestimmtem Molekulargewicht von 400 bis 6000 g/mol auf Basis von Hexandiol-1,6, Butandiol-1,4 oder deren Mischungen handelt.

6. Polyurethanharnstoff nach einem der Ansprüche 1 bis 5, **dadurch gekennzeichnet, dass** er ein zahlenmittleres Molekulargewicht von 1000 bis 100000 g/mol, gemessen in Dimethylacetamid bei 30 °C, aufweist

7. Polyurethanharnstoff nach einem der Ansprüche 1 bis 6, **dadurch gekennzeichnet, dass** er pharmakologische Wirkstoffe enthält.

8. Substrat mit einer darauf aufgebrachten Grundbeschichtung aus einem Polyurethanharnstoff nach einem der Ansprüche 1 bis 7.

9. Substrat nach Anspruch 8, **dadurch gekennzeichnet, dass** auf der Grundbeschichtung eine Deckbeschichtung aus einem Polyurethanharnstoff gemäß einem der Ansprüche 1 bis 6 aufgebracht ist, die sich in ihren chemischen und / oder physikalischen Eigenschaften von der Grundbeschichtung unterscheidet.

10. Substrat nach Anspruch 9, **dadurch gekennzeichnet, dass** die Grundbeschichtung einen pharmakologischen Wirkstoff enthält.

11. Substrat nach einem der Ansprüche 9 oder 10, **dadurch gekennzeichnet, dass** die Deckbeschichtung wirkstofffrei ist.

12. Substrat nach Anspruch 9 bis 11, **dadurch gekennzeichnet, dass** die Grundbeschichtung eine Schichtdicke von 5 bis 20 µm und / oder die Deckbeschichtung eine Schichtdicke von 0,5 bis 10 µm hat.

13. Substrat nach einem der Ansprüche 9 bis 12, **dadurch gekennzeichnet, dass** es ein medizinischer Artikel, bevorzugt ein implantierbar Artikel und insbesondere bevorzugt ein Stent ist.

14. Schichtaufbau umfassend wenigstens eine wirkstoffhaltige Schicht aus einem Polyurethanharnstoff nach Anspruch 7 und wenigstens eine wirkstofffreie Schicht aus einem Polyurethanharnstoff gemäß einem der Ansprüche 1 bis 6.

15. Verfahren zum Beschichten eines Substrats, bei dem auf das Substrat wenigstens eine Schicht aus einem Polyurethanharnstoff nach einem der Ansprüche 1 bis 7 aufgebracht wird.

16. Verfahren nach Anspruch 15, **dadurch gekennzeichnet, dass** auf das Substrat eine Grundbeschichtung aus einem wirkstoffhaltigen Polyurethanharnstoff gemäß Anspruch 7 und auf die Grundbeschichtung eine Deckbeschichtung aus einem wirkstofffreien Polyurethanharnstoff gemäß einem der Ansprüche 1 bis 6 aufgebracht wird.

## Claims

1. Polyurethane urea which has structural units of the formula (I) 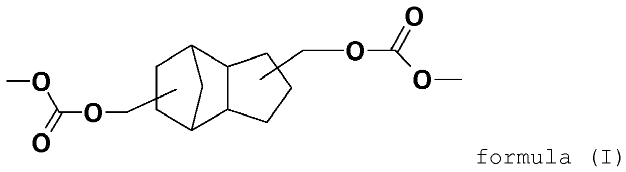 and is not terminated with at least one copolymer unit of polyethylene oxide and polypropylene oxide.

2. Polyurethane urea according to Claim 1, **characterized in that** it is based on a polycarbonate polyol component which preferably has an average hydroxyl functionality of 1.7 to 2.3.

3. Polyurethane urea according to Claim 2, **characterized in that** the polycarbonate polyol component comprises polycarbonate polyols a1) which are obtainable by reaction of carbonic acid derivatives with difunctional alcohols of the formula (II)

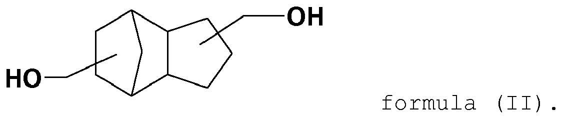

4. Polyurethane urea according to Claim 3, **characterized in that** the polycarbonate polyol component further to the polycarbonate polyols a1) comprises other polycarbonate polyols a2).

5. Polyurethane urea according to Claim 4, **characterized in that** the polycarbonate polyols a2) comprise compounds which have an average hydroxyl functionality of 1.7 to 2.3 and a molecular weight, as determined by the OH number of 400 to 6000 g/mol and are based on hexane-1,6-diol, butane-1,4-diol or mixtures thereof.

6. Polyurethane urea according to any of Claims 1 to 5, **characterized in that** it has a number-average molecular weight of 1000 to 100 000 g/mol as measured in dimethylacetamide at 30°C.

7. Polyurethane urea according to any of Claims 1 to 6, **characterized in that** it comprises active pharmacological ingredients.

8. Substrate having applied thereon a basecoat comprising a polyurethane urea according to any of Claims 1 to 7.

9. Substrate according to Claim 8, **characterized in that** on the basecoat a topcoat is applied which comprises a polyurethane urea according to any of Claims 1 to 6, and which differs in its chemical and/or physical properties from the basecoat.

10. Substrate according to Claim 9, **characterized in that** the basecoat comprises an active pharmacological ingredient.

11. Substrate according to either of Claims 9 and 10, **characterized in that** the topcoat is free from active ingredient.

12. Substrate according to Claim 9 to 11, **characterized in that** the basecoat has a coat thickness of 5 to 20 µm and/or the topcoat has a coat thickness of 0.5 to 10 µm.

13. Substrate according to any of Claims 9 to 12, **characterized in that** it is a medical article, preferably an implantable article, and more preferably a stent.

14. Layer structure comprising at least one active ingredient-containing layer comprising a polyurethane urea according to Claim 7 and at least one active ingredient-free layer comprising a polyurethane urea according to any of Claims 1 to 6.

15. Method for coating a substrate, wherein at least one layer comprising a polyurethane urea according to any of Claims 1 to 7 is applied to the substrate.

16. Method according to Claim 15, **characterized in that** a basecoat comprising an active ingredient-containing polyurethane urea according to Claim 7 is applied to the substrate, and a topcoat comprising an active ingredient-free polyurethane urea according to any of Claims 1 to 6 is applied to the basecoat.

## Revendications

1. Polyuréthane-urée qui présente des unités de structure de formule (I) 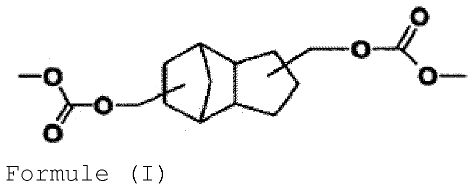 et qui n'est pas terminée par au moins une unité de copolymère de poly(oxyde d'éthylène) et de poly(oxyde de propylène)

2. Polyuréthane-urée selon la revendication 1, **caractérisée en ce qu'**elle est à base d'un composant polycarbonatepolyol, qui présente de préférence une fonctionnalité hydroxyle moyenne de 1,7 à 2,3.

3. Polyuréthane-urée selon la revendication 2, **caractérisée en ce que** le composant polycarbonatepolyol comprend des polycarbonatepolyols a1) qui peuvent être obtenus par transformation de dérivés d'acide carbonique avec des alcools difonctionnels de formule (II)

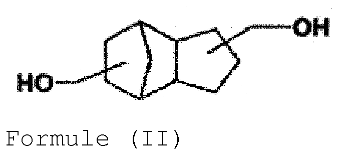

4. Polyuréthane-urée selon la revendication 3, **caractérisée en ce que** le composant polycarbonatepolyol comprend, outre les polycarbonatepolyols a1), d'autres polycarbonatepolyols a2).

5. Polyuréthane-urée selon la revendication 4, **caractérisée en ce qu'**il s'agit, pour les polycarbonatepolyols a2), de composés présentant une fonctionnalité hydroxyle moyenne de 1,7 à 2,3 et un poids moléculaire déterminé par l'indice d'OH de 400 à 6000 g/mole à base d'hexanediol-1,6, de butanediol-1,4 ou de leurs mélanges.

6. Polyuréthane-urée selon l'une quelconque des revendications 1 à 5, **caractérisée en ce qu'**elle présente un poids moléculaire numérique moyen de 1000 à 100 000 g/mole, mesuré dans du diméthylacétamide à 30°C.

7. Polyuréthane-urée selon l'une quelconque des revendications 1 à 6, **caractérisée en ce qu'**elle contient des substances actives pharmacologiques.

8. Substrat sur lequel est appliqué un revêtement de base en une polyuréthane-urée selon l'une quelconque des revendications 1 à 7.

9. Substrat selon la revendication 8, **caractérisé en ce qu'**un revêtement de recouvrement en une polyuréthane-urée selon l'une quelconque des revendications 1 à 6, qui se distingue par ses propriétés chimiques et/ou physiques du revêtement de base, est appliqué sur le revêtement de base.

10. Substrat selon la revendication 9, **caractérisé en ce que** le revêtement de base contient une substance active pharmacologique.

11. Substrat selon l'une quelconque des revendications 9 ou 10, **caractérisé en ce que** le revêtement de base est exempt de substances actives.

12. Substrat selon la revendication 9 à 11, **caractérisé en ce que** le revêtement de base présente une épaisseur de couche de 5 à 20 µm et/ou le revêtement de recouvrement présente une épaisseur de couche de 0,5 à 10 µm.

13. Substrat selon l'une quelconque des revendications 9 à 12, **caractérisé en ce qu'**il s'agit d'un objet médical, de préférence d'un objet médical implantable et en particulier de préférence d'une endoprothèse.

14. Structure à couches comprenant au moins une couche contenant une/des substance(s) active(s) en une polyuréthane-urée selon la revendication 7 et au moins une couche exempte de substances actives en une polyuréthane-urée selon l'une quelconque des revendications 1 à 6.

15. Procédé pour le revêtement d'un substrat, dans lequel au moins une couche en une polyuréthane-urée selon l'une quelconque des revendications 1 à 7 est appliquée sur un substrat.

16. Procédé selon la revendication 15, **caractérisé en ce qu'**un revêtement de base en une polyuréthane-urée contenant une/des substance(s) active(s) selon la revendication 7 est appliquée sur le substrat et un revêtement de recouvrement en une polyuréthane-urée exempte de substances actives selon l'une quelconque des revendications 1 à 6 est appliqué sur le revêtement de base.
